# EUROPEAN PATENT APPLICATION

(11) **EP 4 442 840 A1**
(43) Date of publication of application: **09.10.2024**
(21) Application number: 22893136.6
(22) Date of filing: 08.11.2022
(51) Int. Cl.: C12Q 1/6886, G01N 33/574, A61P 35/00, G01N 33/545, G01N 33/68

(54) **HIGH ATP-AFFINITY PROTEIN AS THERAPEUTIC TARGET FOR INTRACTABLE CANCER MOLECULAR SUBTYPES AND INHIBITOR THEREOF**

(30) Priority: 09.11.2021 KR 20210153213; 21.06.2022 KR 20220075597
(71) Applicant: Veraverse Co., Ltd., Seoul 08502 (KR)
(72) Inventor: CHEONG, Jae Ho, Seoul 06601 (KR); JHE, Yoo-Lim, Seoul 08089 (KR)
(74) Representative: BCKIP Part mbB
(86) International application number: PCT/KR2022/017407
(87) International publication number: WO 2023/085716

(57) **Abstract**

The present invention relates to a therapeutic target for stem-like, epithelial-to-mesenchymal transition and mesenchymal (SEM) cancer subunits and an inhibitor thereof. In the present invention, on the basis of the fact that the intracellular concentration of ATP is high in SEM cancer subunits, a preparation for the prevention or treatment of gastric cancer can be effectively screened for by measuring the mRNA level of a gene encoding an ATP affinity protein or the expression level of the ATP affinity protein, information for predicting and diagnosing gastric cancer can be provided, a kit for diagnosing gastric cancer or predicting the prognosis thereof is provided, and a composition for diagnosing gastric cancer or predicting the prognosis thereof can be effectively provided.

## Description

### TECHNICAL FIELD

The present invention relates to therapeutic targets for Stem-Like, Epithelial-to-Mesenchymal Transition and Mesenchymal Subtype cancer and inhibitors thereof.

### BACKROUND ART

Gastric cancer, in particular, exhibits a high frequency of occurrence in Asia and is a major cause of cancer-related death. It is assumed that 16.2% of cancer patients in Korea (20.3% of male cancer patients and 11.2% of female cancer patients) are gastric cancer patients. The symptoms of gastric cancer show various aspects from the case where there is no symptoms at all to extremely severe pain, the symptoms of gastric cancer do not have specific characteristics, but are general digestive symptoms, and there is no symptoms in the early stage of gastric cancer, Even though there is a symptom, it may be very moderate such as indigestion or uncomfortableness at the upper abdomen and thus, it is quite easy for most people to disregard them and thereby, increasing the mortality rate of gastric cancer.

Recently, it has been confirmed that molecular subtypes can be classified into intestinal, stem-like, mixed stromal, and inflammatory subtypes according to the expression pattern characteristics of genes in microarray analysis of gastric cancer patient tissues, and as mentioned above, it has been known that intestinal subtype gastric cancer is easier to treat than stem-like, mixed stromal or inflammatory subtype gastric cancers, and especially stem-like subtype is reported to be difficult to treat and to have very poor prognosis.

In addition, cancer stem cells or tumor initiating cells exist in cancer tissues that maintain and regenerate cancer tissues like normal organs, and these cancer stem cells are believed to be involved in the process of cancer as the first onset of cancer, as well as angiogenesis and neovascularization in cancer tissues. In addition, it has been reported that it accelerates cancer cell infiltration and is involved in the regeneration of cancer cells that have decreased after cancer treatment, which has a profound effect on cancer recurrence, metastasis, and resistance to chemotherapy. Therefore, in order to fundamentally diagnose and treat cancer, it is necessary to focus on cancer stem cells, which account for only a small part of cancer tissue, but play a key role in the development and maintenance of cancer, anticancer drug resistance, and recurrence, and a further understanding of cancer stem cells will help in the development of diagnostic and therapeutic markers for early diagnosis.

### DETAILED DESCRIPTION OF THE INVENTION

### Technical Problem

The present disclosure found that most malignant cells in stem-like, epithelial-to-mesenchymal transition and mesenchymal (SEM) subtype cancer use a high bioenergy state, and provide a new treatment target, particularly for the stem-like, epithelial-to-mesenchymal transition and mesenchymal stem subtype cancer.

Accordingly, some embodiments of the present disclosure provide a composition comprising a compound for diagnosing gastric cancer or predicting prognosis, wherein the compound measures mRNA level of a gene encoding the ATP affinity protein or the expression level of an ATP-affinity protein.

Some embodiments of the present invention provide a kit comprising the composition for diagnosing gastric cancer or predicting prognosis.

Some embodiments of the present disclosure provide a method of providing information for predicting and diagnosing gastric cancer comprising: (a) measuring mRNA level of a gene encoding an ATP affinity protein or an expression level of an ATP-affinity protein in a biological sample; and (b) comparing the measurement result from step (a) with the mRNA level of the gene encoding the ATP affinity protein or the expression level of the ATP affinity protein or of the control sample.

Another embodiments of the present disclosure provide a method of screening an agent for preventing or treating gastric cancer comprising: (a) treating gastric cancer cells with a gastric cancer treatment a candidate substance; (b) measuring mRNA level of a gene encoding an ATP affinity protein or an expression level of an ATP-affinity protein in the isolated gastric cancer cells treated with a candidate substance in step (a); (c) determining that the candidate material can be used as an agent for preventing or treating gastric cancer when if the mRNA level of a gene encoding an ATP affinity protein or an expression level of an ATP-affinity protein measured in step (b) is lower than that of the isolated gastric cancer cells in which the candidate material is not treated.

Still another embodiments of the present disclosure provide a pharmaceutical composition for preventing or treating gastric cancer comprising a substance targeting epithelial-to-mesenchymal transition (EMT) as an active ingredient.

However, the technical tasks to be solved by the present disclosure is not limited to the above-mentioned task, and other challenges not mentioned may be clearly understood by those skilled in the art from the following description.

### Problem Solving Means

Hereinafter, the present disclosure will be described in more detail.

In the present disclosure, the gene encoding ATP affinity protein is one or more selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB and PSMD9, and therefore, a composition for diagnosing gastric cancer or predicting prognosis of the present disclosure, a kit for diagnosing gastric cancer or predicting prognosis, a method for providing information for predicting and diagnosing gastric cancer, and a method for screening an agent for preventing or treating gastric cancer may include a combination of 1, 2, 3, 4, 5, 6, 7, 8, 9, 10, 11, 12, 13, 14, 15, 16, 17, 18 or 19 types of genes selected from the above genes. In the present disclosure, "a method or a composition for measuring the level of mRNA" refers to a method or a composition used therefor for measuring the level of mRNA transcribed from the gene in order to confirm whether 19 genes of the present disclosure are expressed in a sample. Specifically, the method or the composition includes quantified real time PCR(RT-PCR), Competitive RT-PCR, real time quantitative RT-PCR, RPA; RNase protection assay, Northern blotting, DNA chip analysis assay, or the like or a primer or probe capable of specifically binding to a target gene used therefor, but not limited thereto.

In the present disclosure, "a method or a composition for measuring a level of a protein" refers to a method or a composition for measuring an expression level of a protein encoded by 19 genes of the present disclosure included in a sample. The proteins encoded by the 19 genes includes Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB and 26S proteasome non-ATPase regulatory subunit 9. Specifically, the composition for measuring the level of the protein may include an antibody specific to the protein, or an aptamer. Specifically, the method or the composition may include western blotting, an enzyme-linked immunosorbent assay (ELISA), a radioimmunoassay (RIA), a radioimmunodiffusion, Ouchattery immunodiffusion method, a rocket immunoelectrophoresis, immunohistochemical staining, Immunoprecipitation assay, Complement Fixation Assay, Immunofluorescence, Immunochromatography, fluorescence-activated cell sorting analysis (FACS), protein chip technology assay, and an antibody used therefor, but is not limited thereto.

In the present disclosure, in order to predict the diagnosis or prognosis of cancer or gastric cancer, a kit may be used for the purpose of measuring the expression level of the above-mentioned Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB, and 26S proteasome non-ATPase regulatory subunit 9

The kit of the present disclosure is a reverse transcription polymerase chain reaction (RT-PCR) kit, a DNA chip kit, an enzyme linked immunosorbent assay (ELISA) kit, a protein chip kit, a rapid kit, or a multiple reaction monitoring (MRM) kit, but is not limited thereto.

Specifically, the kit for measuring the expression level of mRNA of the gene may be a kit including essential elements required to perform RT-PCR. RT-PCR kit may contain, in addition to each pair of primers specific to the gene, test tubes or other suitable containers, reaction buffers (various pH and magnesium concentrations), enzymes such as deoxynucleotides (dNTPs), Taq-polymerase, and reverse transcriptase, DNase or RNase inhibitor, DEPC-water, sterile water, etc.. In addition, a primer pair specific to a gene used as a quantitative control may be included.

In addition, the kit of the present disclosure may include essential elements required to perform a DNA chip analysis assay. A kit for DNA chip analysis assay may include a chip where a gene or cDNA corresponding to the gene or a fragment thereof is attached as a probe, and a reagent, a composition, an enzyme, and the like for preparing a fluorescent probe. In addition, the substrate may include cDNA corresponding to a quantitative control gene or a fragment thereof.

In addition, the kit of the present invention may be a protein chip assay kit for measuring the level of a protein encoded from the gene, including but not specifically limited to, a reference for immunological detection of the antibody, a suitable buffer solution, a secondary antibody labeled with chromogenic enzymes or fluorescent substances, a chromogenic substrate, etc. The above substrates may be used, including but not limited to, nitrocellulose membranes, 96-well plates synthesized with polyvinyl resin, 96-well plates synthesized with polystyrene resin, and glass slide glasses, and chromatic enzymes may be used but not limited to peroxidase, alkaline phosphatase, fluorescent substances are not specifically limited to this, but may be FITC, RITC, etc., and chromogenic substrate fluids are not specifically limited to this, but ABTS (2,2'-azino-bis(3-ethylbenzothiazoline-6-sulfonic acid)) or OPD (o-phenylenediamine), It can be TMB (tetramethyl benzidine).

An embodiment of the present invention provides a method of providing information for predicting and diagnosing gastric cancer comprising: (a) measuring mRNA level of a gene encoding an ATP affinity protein or an expression level of an ATP-affinity protein in a biological sample; and (b) comparing the measurement result from step (a) with the mRNA level of the gene encoding the ATP affinity protein or the expression level of the ATP affinity protein or of the control sample.

As used herein, the term "entity" refers to an object for diagnosing gastric cancer or predicting the prognosis of gastric cancer. In this case, the individual may be included without limitation as long as it is an animal in which gastric cancer such as canine, horse, cow, butter, chlorine, rabbit, gum, duck, and stomach can be developed, including humans.

As used herein, the term "sample" includes, but is not limited to, tissues, cells, whole blood, serum, plasma, saliva, sputum, cerebrospinal fluid, or urine separated from an individual suspected of having gastric cancer pathogenesis, and specifically, the tissue may be a gastric mucosa (GM) or an IM tissue, but is not limited thereto.

As used herein, the term "normal entity" refers to an entity that is not diagnosed with gastric cancer, and it is possible to accurately predict the onset of gastric cancer or the prognosis of gastric cancer in an individual suspected of having gastric cancer pathogenesis by measuring and comparing the expression level of the mRNA of the gene or the expression level of a protein expressed therefrom in a sample isolated from an individual to predict the diagnosis or prognosis of gastric cancer and a sample isolated from a normal individual.

Another embodiments of the present disclosure provide an device for predicting and diagnosing gastric cancer comprising: a measuring unit that measures the mRNA level of a gene encoding an ATP affinity protein present in a biological sample or the expression level of an ATP affinity protein; a comparison unit comparing the measurement results with the mRNA level of the gene encoding the ATP affinity protein or the expression level of the ATP affinity protein in the control sample.

The diagnostic device of the present disclosure comprises: a measuring unit to measure the expression level of one or more genes or proteins encoded thereby selected from a group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB, and PSMD9 from a biological sample obtained from a subject; and a computation unit that calculates a score value from the expression level of the gene measured or the protein encoded thereby in the measured region of the measurement.

The computation unit of the diagnosis device of the present disclosure performs a function of calculating a score value with an average of log intensity of the expression level of the gene or protein measured by the measuring unit.

The prognosis of cancer treatment of the target subject can be predicted from the score value derived from the computation unit of the diagnosis device of the present disclosure. More specifically, the computation unit may generate and classify information on the prognosis prediction of cancer according to the value of the derived SMC score to determine the target subject having a bad prognosis with a malignant cancer having the characteristics of stem-like cancer.

In one embodiment of the present disclosure, when the calculated score value is greater than or equal to 5 to 15, preferably, 10 to 12, or more preferably 11 or more, it is possible to further include an output unit for predicting and outputting that the prognosis of cancer patients will not be good due to high possibility of developing or developing malignant cancer including cancer stem cells.

In another embodiment of the present invention, when the calculated SMC score value is between 5 to 15, preferably 10 to 12, or more preferably less than 11, it is possible to predict and output a treatment prognosis of a cancer patient.

The diagnosis device of the present invention may further include an output unit for outputting the prognosis of the cancer of the target predicted by the computation unit.

Another embodiment of the present invention provides a method of screening an agent for preventing or treating gastric cancer comprising: (a) treating gastric cancer cells with a gastric cancer treatment a candidate substance; (b) measuring mRNA level of a gene encoding an ATP affinity protein or an expression level of an ATP-affinity protein in the isolated gastric cancer cells treated with a candidate substance in step (a); (c) determining that the candidate material can be used as an agent for preventing or treating gastric cancer when if the mRNA level of a gene encoding an ATP affinity protein or an expression level of an ATP-affinity protein measured in step (b) is lower than that of the isolated gastric cancer cells in which the candidate material is not treated.

The protein used in the screening method of the present invention may have a form that is displayed on the surface of a cell, a form that is displayed on the surface of a virus (e.g. a bacteriophage), an isolated form, or a purified form. When a protein expressed on the surface of a cell or a surface of a virus is used, the cell or virus is preferably immobilized on a solid substrate for rapid or automation of screening. In addition, it is preferable that the isolated or purified form of protein is immobilized on a solid substrate. Usable substrates include any substrate that may be commonly used in the art, including, but not limited to, hydrocarbon polymers such as polystyrene and polypropylene, glass, metals, or gels. The solid substrate may be provided in the form of a dipstick, a microtiter plate, particles (e.g. beads), an affinity column, and an immunobaline film (e.g. a polyvinylidene fluoride film). Most preferably, the solid substrate is a microtiter plate.

The screening method of the present invention can be carried out in various ways, and in particular, can be carried out in a high throughput manner according to various binding assays known in the art.

In the screening method of the present invention, the test material or the proteins may be labeled with a detectable label. For example, the detectable label may be a chemical label (e.g. biotin), an enzyme label (e.g. a horseradish peroxidase, an alkaline phosphatase, a peroxidase, a luciferase, a beta-galactosidase and a beta-glucosidase), a radioactivity marker (e.g. C-14, I-125, P-32, and S-35), a fluorescent label (e.g. coumarin, fluorescein, FITC alkene, rhodamine 6G BAL), Rhodamine B BAL, Tamra^{®} (6-carboxy-tetramethyl-rhodamine), Cy-3 alkene, Cy -5 SCF, Texas Buffered Butter, Alexa Flufluor^{®}, DAPI^{®} (4,6-diamidino -2 phenylindole), Hex^{®}, Tet^{®}, Dabsyl^{®} and FAM^{®}, luminescent labeling, Chemiluminescent labeling, fluorescence resonance energy transfer (FRET) labeling, or metal labeling (e.g. gold and silver).

When the detectable label uses a labeled protein or test material, whether the protein is bound to the test material may be analyzed by detecting a signal coming from the marker. For example, when alkaline phosphatase is used as a marker, bromochloroindolyl phosphate (BCIP^{®}), nitro blue tetrazolium (NBT^{®}), Signals are detected using chromogenic substrates such as Naphthol-AS-B1 Fix-Phosphate and Enhanced Chemiluminescence (ECF). When a horse radish peroxidase is used as a label, chloronaphthol, aminoethylcarbazole, diaminobenzidine, D - luciferase, luciferase (bis-N alpha-methylacridinium nitrate), resorcinol benzyl ether, luminol, rock flex red reagent (10-acetyl-3,7-dihydroxyphenoxazine), a signal is detected by using a substrate such as p-phenylenediamine (HYR), tetramethylbenzidine (TMB), 2,2 '-azine di [3-ethylbenzthiazoline sulfonate] (ABTS), o-phenylenediamine (OPD), and naphthol/pyronin.

Alternatively, whether the test substance is bound to a protein May be analyzed without labeling of the interaction. For example, a microphysiometer may be used to analyze whether the test material binds to QP-C. The microphysiometer is an analytical tool that uses a light-addressable potentiometric sensor (LAPS) to measure the rate at which the cell acidifies its environment. The change in the acidification rate may be used as an indicator for the binding between the test material and the QP-C residue.

The binding ability with the protein of the test material may be analyzed using real-time bimolecular interaction analysis (BIA). The BIA genome is a technology for analyzing specific interactions in real time and can be carried out without labeling of interactants (e.g. BIAcore^{™}). The change in surface plasmon resonance (SPR) may be used as an indicator for real-time reaction between molecules. In addition, the screening method of the present invention may be performed according to a two-hybrid analysis or three-hybrid analysis method. In this case, the protein may be used as a bait protein. According to this method, a substance binding to the protein, particularly a protein, can be screened. A two-hybrid system is based on a module characteristic of a transcription factor consisting of a dividable DNA fix-binding and activation domain. Simply, the analysis method uses two DNA constructs.

In addition to the above-described single compound or mixture of compounds, the reagent used in the present invention includes peptides, antibodies, peptide aptamers, AdNectin, affibody, Avimer, or Kunitz domain.

The cancer drugs identified by the screening method of the present invention not only target general cancer cells that account for the majority of cancer tissue, but also targets cancer or cancer stem cells that occupy only a small part of the cancer tissue but play a key role in the development, maintenance, and recurrence of cancer, so that it is possible to treat cancer or cancer fundamentally. In addition, the screening method of the present invention can identify cancer therapeutics that act selectively effective on intractable SEM subtype cancers.

In the present invention, "gastric cancer" refers to cancer that occurs in the stomach, and gastric adenocarcinoma, which accounts for the majority of gastric cancers, arises from glandular cells (gland cells) of the gastric mucosa, and can be further divided into several types according to the shape observed under the microscope. Other cancers include lymphomas that originate in lymphoid tissue, interstitial tumors that develop in the nerve and muscle tissue of the stomach, sarcomas (malignant tumors that originate from non-epithelial tissue), and neuroendocrine carcinomas that secrete hormones. In the invention, gastric cancer is preferably a stem-like subtype and/or a mixed stromal subtype, more preferably a stem-like subtype.

Gastric cancer can be classified according to its molecular subtype. For example, mRNA expression at the whole genome level can be examined using microarray techniques in gastric cancer samples, subtypes inherent in gastric cancer can be identified through cluster analysis, and genes specific to each subtype can be selected through statistical validation. Based on the amount of gene expression selected, the subtype with high gene expression characteristic of epithelial cells can be classified into the intestinal subtype, the subtype with high expression of genes derived from developmental stage signaling (EMT) and stroma can be classified into the stem-like subtype, the mixed stromal subtype that expresses both long and stem characteristics, and the inflammatory subtype with high expression of immunomodulatory-related genes. Each subtype was found to have differential characteristics associated with well-defined clinical and pathohistological findings.

Another embodiment of the present invention relates to a method of prevention, improvement, or treatment of cancer, which involves administering an effective dose of the composition of the present invention to a subject in need thereof.

In the present invention, the term "subject" refers to an entity that has been developed with cancer or has a high possibility of developing cancer.

On the other hand, in the present invention, "prevention" may include, without limitation, any act that blocks the symptoms of a disease or suppresses or delays the symptoms by using the pharmaceutical composition of the present invention.

In the present invention, the term "inhibition" refers to any act that inhibits the cancer metastasis by administration of a composition according to the present invention.

In addition, in the present invention, "diagnosis" includes determining an entity's susceptibility to a particular disease or disease, determining whether an entity currently has a particular disease or ailment, or monitoring the condition of an entity to provide information about therapeutic efficacy. For the purpose of the present invention, a diagnosis is to determine the occurrence of cancer or gastric cancer or the stage of development of gastric cancer.

In addition, in the present invention, "prognosis" means the expectation of medical intrigue (e.g., long-term viability, disease-free survival rate, etc.), and includes a positive prognosis (positive prognosis) or a negative prognosis (negative prognosis), the negative prognosis includes disease progression or mortality such as recurrence, tumor growth, metastasis, drug resistance, etc., and the positive prognosis includes remission of disease such as absence of disease, improvement or stabilization of disease such as tumor regression. For the purpose of the present invention, the prognosis is to predict in advance the course of the disease (progression of the disease, improvement, recurrence of gastric cancer, tumor growth, drug resistance) of a patient diagnosed with cancer or gastric cancer.

In addition, in the present invention, "treatment" may include, without limitation, any act that improves or benefits the symptoms of a disease by using the pharmaceutical composition of the present invention.

On the other hand, but not limited to, the method of prevention or treatment of said disease may be combination therapy, which further includes the administration of compounds or substances that have therapeutic activity for one or more diseases.

In the present invention, the "concomitant or combination" should be understood to indicate simultaneous, individual or sequential administration. If the dosing is sequential or individual, the interval between the administration of the secondary component should be such as not to lose the beneficial effect of the concomitant

In the present invention, the pharmaceutical composition may be a capsule, a tablet, a granule, an injection, an ointment, a powder, or a beverage, and the pharmaceutical composition may be used for a human subject.

In the present invention, the pharmaceutical composition is not limited to these, but each may be formulated and used in the form of an acid, granule, capsule, tablet, aqueous suspension, etc., in the form of an oral formulation, topical agent, suppository, and sterile injection solution in accordance with the usual method. The pharmaceutical composition of the present invention may include a pharmaceutically acceptable carrier, or further include excipients, binders, synovies, disintegrators, excipients, solubilizers, dispersants, stabilizers, suspensions, colors, fragrances, etc., when administered orally, and in the case of injections, buffers, preservatives, freeze-free agents, solubilizers, isotonic agents, stabilizers, etc. In the case of topical administration, bases, excipients, lubricants, preservatives, etc. can be further used. The formulation of the pharmaceutical composition of the present invention may be prepared in a variety of ways by mixing with a pharmaceutically acceptable carrier or excipients as described above. For example, when administered orally, it can be prepared in the form of tablets, trochi, capsules, elixir, suspension, syrup, wafers, etc., and in the case of injections, it can be prepared in the form of unit-dose ampoules or multi-doses. It can be formulated as others, solutions, suspensions, tablets, capsules, extended-release formulations, etc.

Examples of carriers, excipients, and diluents suitable for formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, mineral oil, and the like. In addition, the compound may further include a filler, an anticoagulant, a lubricant, a wetting agent, a fragrance, an emulsifier, a preservative, and the like.

The administration routes of the pharmaceutical composition of the present invention are not limited to these but include intra-oral, intravenously, intramuscular, intramuscular, intramedullary, intranasal, intestinal, transdermal, subcutaneous, intraperitoneal, intranasal, intestinal, topical, sublingual or rectal. Oral or parenteral administration is preferred. It is more preferred to be administered directly to the eye.

In the present invention, the "parenteral" may be directly administered to the eye, subcutaneous, intradermal, intravenously, intramuscular, intra-joint, synovial fluid, sternum, intrathecal, in-situ, and intramuscular injection or injection. The pharmaceutical composition of the present invention can also be administered in the form of a suppository for rectal administration. It is preferred to be administered directly to the eye.

The "regime and dose" of the composition of the present invention may vary in various ways according to various factors including the activity of a particular compound used, age, body weight, general health, gender, formula, administration time, administration route, discharge rate, drug compounding and prevention or treatment of certain diseases to be prevented or treated, and the dosage of the pharmaceutical composition may vary depending on the condition, weight, degree of disease, dosage form, administration route and period of the patient, but may be suitably selected by one of ordinary skill in the art, and 0.0001 to 50 mg/kg or 0. It can be administered at 001 to 50 mg/kg. The administration may be administered once a day or several times. The dosage is not intended to limit the scope of the present invention in any aspect. The pharmaceutical composition according to the present invention may be formulated into a pill, a sugar tablet, a capsule, a liquid, a gel, a syrup, a slurry, and a suspension.

Some embodiments of the present invention provide a composition for diagnosing gastric cancer or predicting a prognosis comprising: a substance for measuring the expression level of an ATP affinity protein or a gene encoding an ATP affinity protein.

In one embodiment of the present invention, the gene encoding the ATP affinity protein is selected from mRNA of a gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB, PSMD9, and a mixture thereof.

In another embodiment of the present invention, the ATP affinity protein is selected from Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB, 26S proteasome non-ATPase regulatory subunit 9, or a mixture thereof.

According to another embodiment of the present invention, the level of the gene encoding the ATP affinity protein is measured by reverse transcription polymerase chain reaction (RT-PCR), competitive RT-PCR, real-time quantitative RT-PCR, RNase protection method, Nothern blotting, DNA chip technology assay, Methylated DNA binding domain sequencing (MBD-seq) assay, or Reduced representation bisulfite sequencing (RRBS) assay.

According to another embodiment of the present invention, the expression level of the ATP affinity protein may be measured by Western blotting, enzyme-linked immunosorbent assay (ELISA), radioimmunoassay (RIA), radial immunodiffusion assay, Ouchterlony immunodiffusion assay, rocket immune-electrophoresis assay, immunohistochemical staining, immunoprecipitation assay, complement Fixation Assay, immunofluorescence, immunochromatography, fluorescence-activated cell sorting (FACS) analysis, protein chip technology assay, or a mixture thereof.

Another embodiment of the present invention provides a composition for diagnosis of the gastric cancer is a composition for diagnosing or predicting a prognosis of treatment of a stem-like, epithelial-to-mesenchymal transition and mesenchymal subunit gastric cancer gastric cancer.

Another embodiment of the present invention provides a kit for diagnosing gastric cancer or predicting prognosis, comprising the above composition.

Some embodiment of the present invention provide the kit provides a kit for diagnosing gastric cancer or predicting prognosis comprising: a reverse transcription polymerase chain reaction (RT-PCR) kit, a DNA chip kit, an enzyme-linked immunosorbent assay (ELISA) kit, or a protein chip kit.

According to another embodiment of the present invention, the gastric cancer provides a kit for diagnosing gastric cancer or predicting the prognosis of gastric cancer, which is a stem-like, epithelial-to-mesenchymal transition and mesenchymal subunit gastric cancer.

Some embodiments of the present disclosure provide a method of providing information for predicting and diagnosing gastric cancer comprising: (a) measuring mRNA level of a gene encoding an ATP affinity protein or an expression level of an ATP-affinity protein in a biological sample; and (b) comparing the measurement result from step (a) with the mRNA level of the gene encoding the ATP affinity protein or the expression level of the ATP affinity protein or of the control sample.

In some embodiments of the present invention, the gene encoding the ATP affinity protein is selected from mRNA of at least one gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB, PSMD9, or a mixture thereof.

According to another embodiment of the present invention, the ATP affinity protein is Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB, 26S proteasome non-ATPase regulatory subunit 9, or a mixture thereof.

According to another embodiment of the present invention, the level of the mRNA of the gene encoding the ATP affinity protein is measured by reverse transcription polymerase chain reaction (RT-PCR), competitive RT-PCR, real-time quantitative RT-PCR, RNase protection method, Nothern blotting, DNA chip technology assay, Methylated DNA binding domain sequencing (MBD-seq) assay, or Reduced representation bisulfite sequencing (RRBS) assay.

According to another embodiment of the present invention, the expression level of the ATP affinity protein may be measured by Western blotting, enzyme-linked immunosorbent assay (ELISA), radioimmunoassay (RIA), radial immunodiffusion assay, Ouchterlony immunodiffusion assay, rocket immune-electrophoresis assay, immunohistochemical staining, immunoprecipitation assay, complement Fixation Assay, immunofluorescence, immunochromatography, fluorescence-activated cell sorting (FACS) analysis, protein chip technology assay, or a mixture thereof.

According to another embodiment of the present invention, the gastric cancer is a stem-like, epithelial-to-mesenchymal transition and mesenchymal subunit gastric cancer.

Another embodiments of the present disclosure provide an device for predicting and diagnosing gastric cancer comprising: a measuring unit that measures the mRNA level of a gene encoding an ATP affinity protein present in a biological sample or the expression level of an ATP affinity protein; a comparison unit comparing the measurement results with the mRNA level of the gene encoding the ATP affinity protein or the expression level of the ATP affinity protein in the control sample,
wherein, the gene encoding the ATP affinity protein is selected from mRNA of a gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB, PSMD9, and a mixture thereof;
wherein, the ATP affinity protein is selected from Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB, 26S proteasome non-ATPase regulatory subunit 9, or a mixture thereof;
wherein, the gastric cancer is a stem-like, epithelial-to-mesenchymal transition and mesenchymal subunit gastric cancer.

Another embodiments of the present disclosure provide a method of screening an agent for preventing or treating gastric cancer comprising: (a) treating gastric cancer cells with a gastric cancer treatment a candidate substance; (b) measuring mRNA level of a gene encoding an ATP affinity protein or an expression level of an ATP-affinity protein in the isolated gastric cancer cells treated with a candidate substance in step (a); (c) determining that the candidate material can be used as an agent for preventing or treating gastric cancer when if the mRNA level of a gene encoding an ATP affinity protein or an expression level of an ATP-affinity protein measured in step (b) is lower than that of the isolated gastric cancer cells in which the candidate material is not treated,
wherein, the gene encoding the ATP affinity protein is selected from mRNA of a gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB, PSMD and a mixture thereof;
wherein, the ATP affinity protein is selected from Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB, 26S proteasome non-ATPase regulatory subunit 9, or a mixture thereof;
wherein, the level of the gene encoding the ATP affinity protein is measured by reverse transcription polymerase chain reaction (RT-PCR), competitive RT-PCR, real-time quantitative RT-PCR, RNase protection method, Nothern blotting, DNA chip technology assay, Methylated DNA binding domain sequencing (MBD-seq) assay, or Reduced representation bisulfite sequencing (RRBS) assay;
wherein, the expression level of the ATP affinity protein may be measured by Western blotting, enzyme-linked immunosorbent assay (ELISA), radioimmunoassay (RIA), radial immunodiffusion assay, Ouchterlony immunodiffusion assay, rocket immune-electrophoresis assay, immunohistochemical staining, immunoprecipitation assay, complement Fixation Assay, immunofluorescence, immunochromatography, fluorescence-activated cell sorting (FACS) analysis, protein chip technology assay, or a mixture thereof; and
wherein, the gastric cancer is a stem-like, epithelial-to-mesenchymal transition and mesenchymal subunit gastric cancer.

Another embodiment of the present invention provides a pharmaceutical composition for preventing or treating gastric cancer, comprising a substance targeting epithelial-to-mesenchymal transition (EMT) as an active ingredient,
wherein, the substance is selected from the group consisting of Niclosamide(Niclocide), TAE684(NVP-TAE684), MLN2238, Bortezomib(Velcade), MLN9708, Carfilzomib(PR-171), Crystal violet, Cetylpyridinium Chloride, Alexidine HCl, WP1130, Pyrithione zinc, BAY 11-7082(BAY 11-7821), Dronedarone HCl(Multaq), Penfluridol, Thonzonium Bromide, LDN193189, Obatoclax mesylate(GX15-070), LY2603618(IC-83), Terfenadine, Vorinostat(SAHA), PCI-24781, AR-42(HDAC-42), Belinostat(PXD101), Trichostatin A(TSA), CUDC-101, SB939(Pracinostat), JNJ-26481585, Fenbendazole(Panacur), BKM120(NVP-BKM120), YM155, Mitoxantrone Hydrochloride, Teniposide(Vumon), Doxorubicin(Adiriamycin), Daunorubicin HCl(Daunomycin HCl), Triptolide, PIK-75, Ouabain, Topotecan HCl, Camptothecin, Gemcitabine(Gemzar), Fludarabine Phosphate(Fludara), GSK2126458, AZD77662, Torin 2, INK 128(MLN0128), BEZ235(NVP-BEZ235), Neratinib(HKI-272), AT9283, Danusertib(PHA-739358), Oxibendazole, Nocodazole, BI6727(Volasertib), BI2536, Vincristine, Vinblastine, Epothilone A, Cephalomannine, Paclitaxel(Taxol), Cabazitaxel(Jevtana), SB743921, Ispinesib(SB-715992), APO866(FK866), and a mixture thereof; and
wherein, the gastric cancer is a stem-like, epithelial-to-mesenchymal transition and mesenchymal subunit gastric cancer.

### Effect of the Invention

When mitochondrial function increases, they produce more ATP. The ATP produced in this way binds to numerous proteins that play a pivotal role in most cellular processes, such as metabolism, synthesis, active transport, cell signaling, migration, and cell structure maintenance. There are currently 1,500 known ATP-binding proteins, and when the demand for ATP is increased under stressful conditions and the cells are bioenergetically damaged, ATP must first be used by proteins with vital functions that are important for cellular compatibility. Therefore, these high ATP affinity proteins may be targets for novel cancer therapies in SEM subtype cancers.

Based on an observation that the concentration of ATP in cells is high in SEM (stem-like, epithelial-to-mesenchymal transition and mesenchymal) subtype cancers, the present invention provides effective method for screening substances for preventing and treating gastric cancer, a kit comprising a composition for diagnosing and/or predicting gastric cancer, and a composition therefor by measuring the mRNA level of the gene encoding the ATP affinity protein or the expression level of the ATP affinity protein.

In addition, according to the embodiment of the present invention, it was found that it exhibited a distinct enrichment profile of ATP-affinity protein in SEM (stem-like, epithelial-to-mesenchymal transition and mesenchymal) subtype cancer than in non-SEM cancer. Consequently, inhibition of bioenergy checkpoints for cellular compatibility by targeting proteins with high ATP affinity may provide vulnerability to drug-resistant cancers. Ultimately, according to the present invention, the genetic dependence of SEM subtypes of cancer on ATP affinity proteins could open up new therapeutic opportunities to selectively target clinically refractory cancers for which genomic analysis alone has few pharmaceutical targets.

### BRIEF DESCRIPTION OF THE DRAWINGS

FIG. 1 is a graph showing that the concentration of ATP is high in SEM-based gastric cancer cell lines than non-SEM-based cell lines according to an exemplary embodiment of the present invention.
FIG. 2 shows a process of LC-MS/MS analysis of ATP bound protein labeled with Desthiobiotin-ATP probe.
FIG. 3 shows quality control results of 10 µg of proteins before and after desalination for the accuracy of an experiment during a desalination process in an ATP quick probe labeling process according to an embodiment of the present invention.
FIG. 4 shows the results confirming that protein labeling was performed well as the marker protein was confirmed after the Desthiobiotin-ATP probe was labeled according to an embodiment of the present invention.
FIG. 5 shows a result of confirming that a labeled peptide is eluted as a result of dot blot analysis to identify a Desthiobiotin-labeled protein according to an embodiment of the present invention.
FIG. 6 shows the number of proteins for each cell line as a result of LC-MS/MS chromatography according to an embodiment of the present invention.
FIG. 7 shows a residue diagram separated by proteins found in non-SEM-type gastric cancer cell lines and SEM-based gastric cancer cell lines confirmed in FIG. 7.
FIG. 8 shows a correlation between Fludarabine, Teniposide^{®}, and Epithelial-to-mesenchymal transition (EMT) by targeting a Monoamine Oxidase B (MAOB) protein according to an embodiment of the present invention.
FIG. 9 illustrates the results of the analysis of chemicals as a candidate pharmaceutical compositions for the prevention or treatment of potential gastric cancer in SEM subtype cell lines through CRISPR-Cas9 viability screening according to one embodiment of the present invention.

### EXAMPLES

Hereinafter, the present invention will be described in more detail with reference to the embodiments. It will be obvious to a person skilled in the art that these examples are only for describing the present invention in more detail, and the scope of the present invention is not limited by these examples according to the subject matter of the present invention.

### Example 1. Culture of Gastric Cancer Cell Line for Each Type

### 1. Culture of Gastric Cancer Cell Line

All experiments were performed by obtaining approval of the Institutional National Review Board (IRB) of YonSei University College of Medicine. For the experiment, the human gastric cancer cell line KCTC746VII, MKN45, YCC7, and YCC11 genome were obtained from the American Type Culture Collection (ATCC), and the human gastric cancer cell line AGS, SNU216, YCC7, and YCC11 genome were obtained from Korean Cell Line Bank. SK4, SNUS484, SNU668, and SNU1750 were obtained, and 1% penicillin-streptomycin (Gibco^{®}) was added to RPMI 1640 medium containing 10% FBS according to the guides of the US or Korea cell line, and then cultured in a 37°C 5% CO₂ incubator (HERAcell 150i, Thermo Scientific) before they were used in the experiment.

### 2. Preparation of Non-SEM-Type Gastric Cancer Cell Line and SEM-Type Gastric Cancer Cell Line

Cells were cultured as provided in Example 1.1, AGS, MKN45, SNU216, and YCC7 were prepared as non-SEM type gastric cancer cell lines, and SAS746VII, SK4, SNU484, SNU668, SNU1750, and YCC11 were prepared as the SEM type of gastric cancer cell line.

### Example 2. Comparison of ATP Concentration in Non-SEM-Type Gastric Cancer Cell Line and SEM-Type Gastric Cancer Cell Line

### 1. ATP Assay

The ATP concentration was measured using an ATP protein determination kit (Invitrogen) in order to confirm an energy level difference in a non-SEM lower-type cell line (AGS to YCC7) which is easy to treat by using the gastric cancer cell line for each type prepared in Example 1 and a gastric cancer cell line (HS746T to YCC11). The high ATP concentration can be a measure showing the characteristics of intractable cancer.

After dispensing 8 different concentrations of ATP solution and 10 µg of protein from each cell line in a 96-well white microplate (SPL) for the generation of standard curves, 90 µL standard reaction solution (Invitrogen) was added to block the light, incubated for 15 minutes, and luminescence was measured using a luminometer (EG&G Berthold).

### 2. Statistical Analysis

After subtracting the background luminescence value from the measured value, the value was quantified according to the standard curve, and the predicted ATP concentration of 1 mg of protein was obtained.

### 3. Conclusion

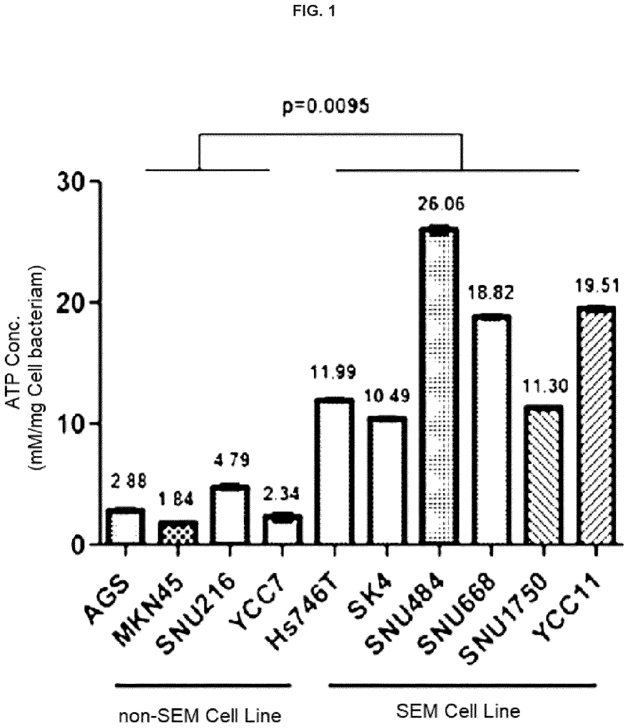

The figures on the drawing are average ± average error range (standard error of the mean; SEM). As shown in Figure 1, the concentration of ATP was at least 5 to 10 times higher in the SEM subtype gastric cancer cell line (Hs746T, SK4, SNU484, SNU668, SNU1750, YCC11) than in the non-SEM subtype cell lines (AGS, MKN45, SNU216, YCC7). This suggests that ATP-affinity proteins are proteins necessary for the survival of cancer cells by acting on the metabolic processes of cancer cells in SEM subtype cancers, suggesting that ATP-affinity proteins can be targets for new cancer therapeutics.

### Example 3. LC-MS/MS Analysis of ATP Protein Binding Protein Labeled with desthiobiotin-ATP Probe

### 1. Basic Principle

The desthiobiotin-ATP probe binds to the pocket of the ATP-binding protein, so that the acyl group present in the desthiobiotin-ATP probe is very close to the lysine sequence of the protein that binds to ATP. Thus, the amino group of lysine of the protein that binds to ATP reacts with the acyl group of the desthiobiotin-ATP probe, resulting in an amide bond between the desthiobiotin-ATP probe and the protein that binds to ATP. Therefore, LC-MS/MS liquid chromatography using tags present in probes covalently linked to proteins that bind to ATP can classify proteins that bind to ATP.

### 2. Detailed Experimental Method

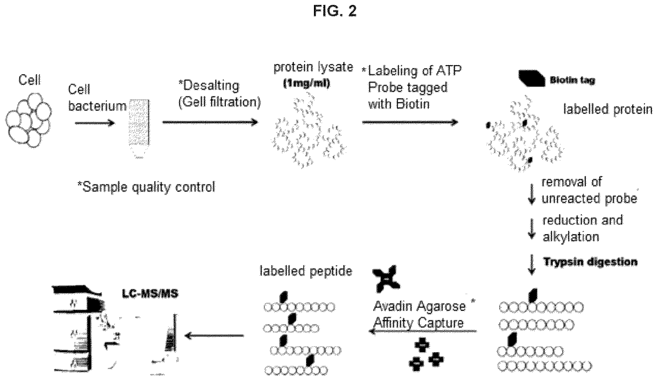

In order to profiling ATP binding protein of each gastric cancer cell line, protein enrichment assay was performed using the ATP probe tagged with dethiobiotin. A schematic test method is shown in FIG. 2.

About 5 × 10⁸ cells were washed twice with cold PBS, and then dissolved in an IP lysis buffer (Thermo Fisher) including Complete protease inhibitor cocktail (Roche) and PhosSTOP phosphatase inhibitor(Roche). After centrifugation the cell lysate at 4°C/16000 g for 30 minutes, the supernatant was subjected to gel filtration using a Zeba spin column (7K MWCO, Pierce) to remove low molecular weight materials therein. Then, Halt protease/phosphatase inhibitor cocktail(Thermo fisher) was added to the samples.

The concentration of protein was quantified by BCA protein assay (Pierce) and diluted with Reaction Buffer (20mM Hepes, pH 7.5, 150mM NaCl, 0.1% Triton-X-100) to set 4 mg/mL. 1 mg of a protein per sample was used, and MgCl₂ was added before the reaction was carried out at room temperature for 1 minute, followed by mixing with dethiobiotin-ATP probe at concentrations 5, 10, 20, 40, 80, and 100 µM and shaking the mixture at room temperature for 10 minutes to label the proteins. After the dethiobiotin-ATP probe labelling, the reduction and alkalization of the labeled protein were performed. A 10 M Urea/IP lysis buffer and 500 mM DTT were added and allowed to react at 65°C for 30 minutes. Thereafter, the mixture was cooled to room temperature, and 1 M iodoacetamide (IAA) was added thereto, and then reacted in a dark room at room temperature for 30 minutes. Next gel filtration was performed using a digestion buffer (2M Urea/20mM Tris, pH8.0), sequencing grade trypsin was added at a ratio of 1:100 and reacted overnight at 37°C to cut the protein into peptides. The labeled peptides were captured at room temperature for 1 hour using the agarose resin. In order to remove the residual peptides, a washing buffer(25mM Tris-HCl, pH 7.4, 150mM NaCl, 1mM EDTA, 1% NP-40, 5% glycerol), PBS, and pure water were used to wash, followed by triturating with 75 µL elution buffer (50% Acetonitrile; ACN, 0.1 % Trifluoroacetic Acid; TFA) three times. The peptide solution was then subjected to a Speed Vacuum System (Savant Instruments Inc.), dissolved in 50 µL of 1% TFA to perform LC-MS/MS (Liquid Efficient Chromatography-Mass Spectrometry/Mass Spectrometry). The database search used the existing protein sequence database through a Mascot algorithm (Matrixscience, USA) as a peptide sequence read by LC-MS/MS, and each variable 1) category: Homo Sapience, 2) fixed modification; cystein residue carbamidomethylation, 3) variable modification; methionine residue oxidation and lysine residue dethiobiotinylation, 4) maximum missed cleavage; 2, 5) MS tolerance; 100 ppm; and 6) MS/MS tolerance; 0.Da to identify proteins.

The type and number of ATP protein binding proteins were confirmed for each cell line as a result of LC-MS/MS using dethiobiotin-ATP probe.

### 3. Three Sample Quality Control

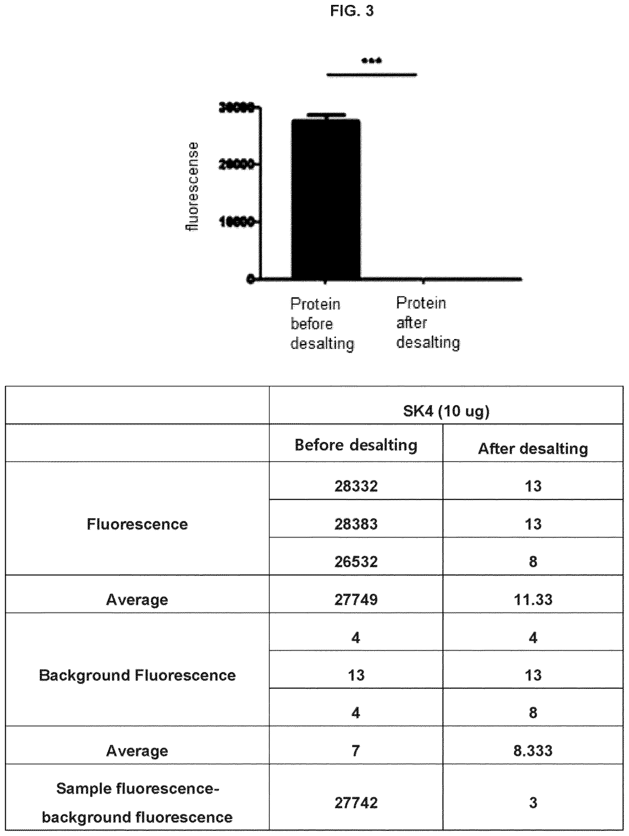

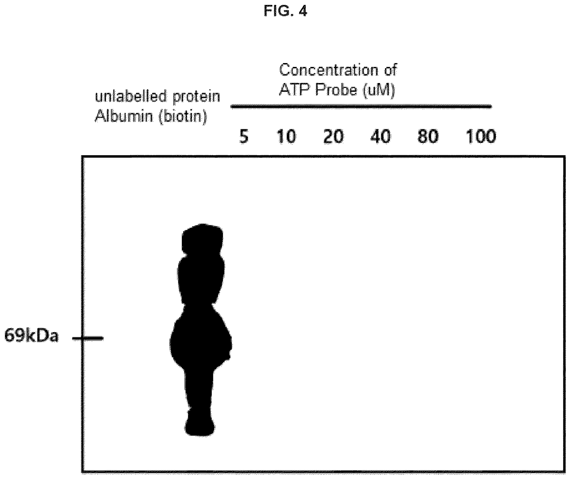

(1) After gel filtration using Zeba spin column (7K MWCO, Pierce), the ATP concentration was measured using an ATP measurement kit (Invitrogen) to confirm removal of ATP. 10 µl of proteins before and after desalting was dispensed into each of a 96-well white microplate (SPL), 90 ul of standard reaction solution (Invitrogen) was added, light was blocked and cultured for 15 minutes, and luminescence was measured using a luminometer (EG&G Berthold). At this time, the background luminescence value was subtracted from the measured value, and the difference in luminescence before and after the desalting is compared, and the results are shown in FIG. 3. As a result, it was confirmed that ATP was removed after desalting when the luminescence was compared.
(2) In order to confirm the desthiobiotin-ATP probe marker protein, 20 ug of protein was loaded after probe labeling to perform western blotting. The streptavidin antibody conjugated with HRP genome was diluted in TBS-0.05% Tween 20 containing 2% BSA in a ratio of 1: 40000, reacted at room temperature for 1 hour, and then an ECL suspension solution (Thermo Fisher) was added and detected using Chemi-Luminescent Image Analyzer (LAS4000, Fujifilm) to show the result in FIG. 4. Based on the result, it was confirmed that as the probe concentration increases, the signal strength of the western blot band increases, and thus the protein labeling was performed well.
(3) A dot blot was performed to identify a labeling peptide captured using an agarose resin after digestion of the desthiobiotin labeled protein with trypsin.

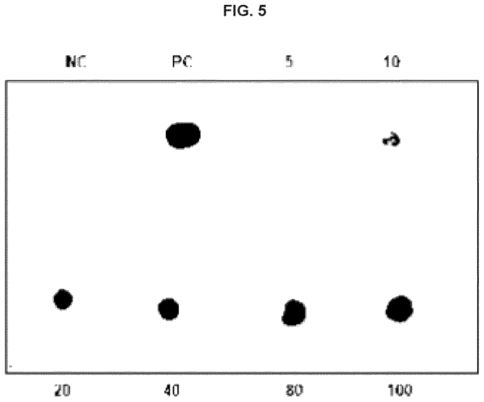

2 uL of the labeled peptide elutes was dispensed into a nitrocellulose membrane (Amersham), dried at room temperature for 1 hour, streptavidin antiboby conjugated with HRP was diluted with TBS-0.05% Tween 20 containing 2% BSA at a ratio of 1: 20000, reacted at room temperature for 1 hour, ECL solution (Thermo fisher) was added, and then subjected to detection using an X-ray film to show the result in FIG. 5. Based on the result, it was confirmed that the labeled peptide was eluted with increasing the probe concentration as the probe concentration increases.

### 4. Conclusion

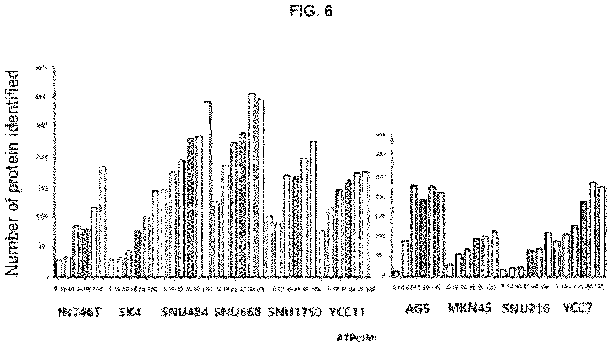

As a result, as shown in FIG. 6, various ATP affinity proteins can be obtained from the non-SEM-type gastric cancer cell line and the SEM-type gastric cancer cell line, respectively, and the specific protein types are shown in Table 1 below.

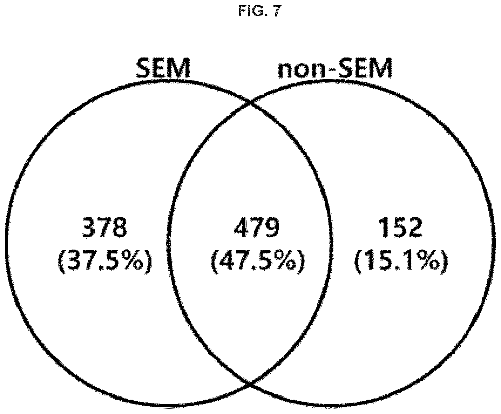

A total of 1009 proteins were confirmed, and the proteins identified in each cell line were analyzed and represented in FIG. 7 as a Venn diagram. In a total of 1009 proteins, the protein identified in the non-SEM subtype gastric cancer cell line was 631 and the protein identified in the SEM subtype gastric cancer cell line was 857. Among them, proteins commonly identified in non-SEM and SEM subtype gastric cancer cell lines were 479.

**TABLE 1**

| Non-SEM Subtype Gastric Cancer Cell Line (631) |
|---|
| CA2,CHEK1,COPS3,CPS1,CSRP1,CYP4F11,DCAF1,DLD,EIF3G,ETFB,FAM83C,FASTK,FKBP10 ,G6PD,GATB,GCLC,GSR,H2AC4,HINT1,HNRNPA0,HNRNPA1L2,HNRNPA2B1,HSP90AA5P,HSP A4,IQSEC2,ITGA4,ITPRIP,KIF23,KYNU,LIG1,MAP3K2,MARK3,MDH2,MSH2,MTMR12,MYH7B,M YOF,NEK7,NQO1,NSUN2,NUDT1,NUP205,OGDH,OR56A4,PAPSS1,PBRM1,PC,PDCD6IP,PDIA 3,PDPK1 ,PFAS,PFKP,PGM2L1,PHKG2,PIKFYVE,PPA1 ,PRAMEF14,PRDX6,PRKCD,PRKDC,PR OCR,PSMD1,PSMD9,PTMA,PTPN21,RAN,RIMS2,ROCK1,RPL29,RRBP1,RRP36,RRS1,SAMD9, SCCPDH,SEC61A1,SF3B6,SGTA,SLC9A3R1,SNCA,SPAG5,SUB1,SUCLA2,TBCD,TMEM232,TO R1B,TP53RK,TPD52,TPM2,TPM4,TRBV11-2,UCHL1,VKORC1,VPS29,ACAP3,AHNAK,AHNAK2,ALDOC,ARHGAP12,ARPP21,ATP5IF1,CAC YBP,CCDC68,DICER1,DTD2,ECI1,ERCC5,HEATR1,ITGB1,LASP1,MAP3K8,MARCKS,MRGBP,O R4K15,POGK,PSMA7,SDHAF4,SERBP1,SNX6,SYNE1,TIAM2,TMSB4X,TPD52L2,UBE2I,YWHAQ .ZRANB1 |

| SEM - Subunit Gastric Cancer Cell Line (857) |
|---|
| 1,XRCC6,XRN2,YARS1,YBX1,YBX3,YWHAB,YWHAE,YWHAZ,ZDHHC8,ZNF106,ZNF212,ZNF34 7,ZNF418,ZNF630 |

### [Example 4] Genetic Dependency Confirmation of ATP-Bound Proteins-Analysis of Genomic Dependency of ATP-Bound Proteins

### 1. Basic Principle

In order to confirm genes encoding a corresponding protein through the ATP affinity protein of the non-SEM type gastric cancer cell line obtained in Example 3 and the stomach cancer cell line of the SEM, the genetic dependency was confirmed in the MAPMAP. Mini MAP ^{®} is the Broad National Institute of MIT Fix/Harvard (Https://iemap. _ Org/Portal/), and in the site, it can be seen that certain genes are essential to survival through knock-out screening using non-gene, which is gene scissors. The lower the dependency score means that the corresponding gene plays an important role in survival in the corresponding cell line

### 2. Genetic Dependence Analysis of Total Cell Lines

A gene having DepMap score - 0.5 or less was selected as a gene that may be treated as a therapeutic target due to a high genetic dependency of a gene having a value of - 0.5 or less on the basis of - 0.5. Non-SEM subtype gastric cancer cell lines, AGS, MKN45, SNU216 and after DepMap score of SEM subtype cell lines Hs746T and SNU668 were obtained, which are available non-SEM subtype gastric cancer cell lines available in the DepMap database, are obtained non-SEM cell lines with gene's DepMap score higher than -0.5 and SEM cell lines with gene's DepMapscore less than -0.5 to extract a total of 25 genes.

### 2. Removal of Normal Gastric Cell (GES -1) and Deduplication

In order to remove normal toxicity filters of normal stomach cells in the MAP genome, genes that may be targets for diagnosis, prevention and treatment of a total of 19 SEM subtype cancer except for the duplicate genes in GES-1, i.e. GART, GPI, HSPA8, PI4KB, PSME2, UBQLN1, were analyzed, and the 19 genes were shown in Table 2 below as dependency scores for each cell line, Table 3 shows the protein expressed by the corresponding gene.

**TABLE 2**

| Gene | Cell line | | | | |
|---|---|---|---|---|---|
| | Non-SEM Subtype Cell Line | | | SEM Subtype Cell Line | |
| | AGS | MKN45 | SNU216 | HS746T | SNU668 |
| ASS1 | -0.42544 | -0.12717 | -0.49589 | -0.33269 | -0.61997 |
| RANBP1 | -0.42024 | -0.25317 | -0.32304 | -0.40195 | -0.56543 |
| HAT1 | -0.12042 | -0.19767 | 0.03069 | -0.03981 | -0.55673 |
| CYB5B | -0.14665 | -0.07314 | -0.13083 | -0.51927 | -0.38912 |
| GANAB | -0.40079 | -0.37345 | -0.40021 | -0.05349 | -0.50057 |
| CAST | -0.19854 | -0.30569 | -0.25432 | -0.44698 | -0.53813 |
| EEF1D | -0.31564 | -0.17606 | -0.21115 | -0.09485 | -0.60652 |
| LIG1 | -0.38159 | -0.42532 | -0.05163 | -0.40179 | -0.5706 |
| PFAS | -0.14391 | -0.44756 | -0.25214 | -0.32370 | -0.68244 |
| PSMD9 | -0.39316 | -0.40107 | -0.37932 | -0.50605 | -0.56145 |
| MTA2 | 0.30066 | -0.23785 | -0.23793 | -0.56731 | -0.58012 |
| MARCKS | -0.22396 | -0.42313 | 0.09197 | -0.21598 | -0.50902 |
| REXO2 | -0.29240 | -0.19861 | -0.2944 | -0.40980 | -0.51803 |
| TPM2 | -0.48756 | -0.46425 | -0.4334 | -0.54367 | -0.33900 |
| YWHAZ | -0.09929 | -0.23304 | -0.43824 | -0.44870 | -0.52129 |

| PNPO | -0.16640 | -0.16875 | -0.17748 | 0.04405 | -0.59051 |
|---|---|---|---|---|---|
| HNRNPA2B1 | -0.03189 | -0.39877 | -0.19111 | -0.98767 | -0.92858 |
| TXNDC17 | -0.24125 | -0.31647 | -0.29967 | -0.98732 | -0.28553 |
| HNRNPAO | -0.11053 | -0.45805 | -0.28298 | -0.71579 | -0.20778 |

**TABLE 3**

| | Gene name | **Entry** | Protein name |
|---|---|---|---|
| 1 | CAST | P20810 | Calpastatin |
| 2 | EEF1D | P29692 | Elongation factor 1-delta |
| 3 | HNRNPA2B1 | P22626 | Heterogeneous nuclear ribonucleoproteins A2/B1 |
| 4 | LIG1 | P18858 | DNA ligase 1 |
| 5 | MARCKS | P29966 | Myristoylated alanine-rich C-kinase substrate |
| 6 | MTA2 | 094776 | Metastasis-associated protein MTA2 |
| 7 | PFAS | 015067 | Phosphoribosylformylglycinamidine synthase |
| 8 | PNPO | Q9NVS9 | Pyridoxine-5'-phosphate oxidase |
| 9 | REXO2 | Q9Y3B8 | Oligoribonuclease, mitochondrial |
| 10 | YWHAZ | P63104 | 14-3-3 protein zeta/delta |
| 11 | HNRNPAO | Q13151 | Heterogeneous nuclear ribonucleoproteins A0 |
| 12 | TPM2 | P07951 | Tropomyosin beta chain |
| 13 | TXNDC17 | Q9BRA2 | Thioredoxin domain-containing protein 17 |
| 14 | ASS1 | P00966 | Argininosuccinate synthase |
| 15 | RANBP1 | P43487 | Ran-specific GTPase-activating protein |
| 16 | HAT1 | 014929 | Histone acetyltransferase type B catalytic subunit |
| 17 | CYB5B | 043169 | Cytochrome b5 type B |
| 18 | GANAB | Q14697 | Neutral alpha-glucosidase AB |
| 19 | PSMD9 | 000233 | 26S proteasome non-ATPase regulatory subunit 9 |

### 3. Correlation Review According to Experimental Results of CRISPR/Cusp9

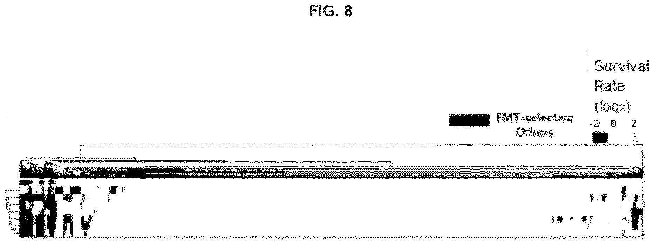

In particular, the correlation of the MAOB protein (monoamine oxidase B ') protein with in silico is shown in FIG. 8.

### 4. Screening of Composition for Diagnosis, Prevention and Treatment of 62 SEM-Specific Gastric Cancer

An FDA-approved small-molecule pharmacological compound (# L1300) and an anticancer compound for research (# L2000) were purchased from Selecckchem and a 10 mM solution was prepared in DMSO. A total of 1,345 compounds were tested the cell viability of the seven EMT gastric cancer cell lines.

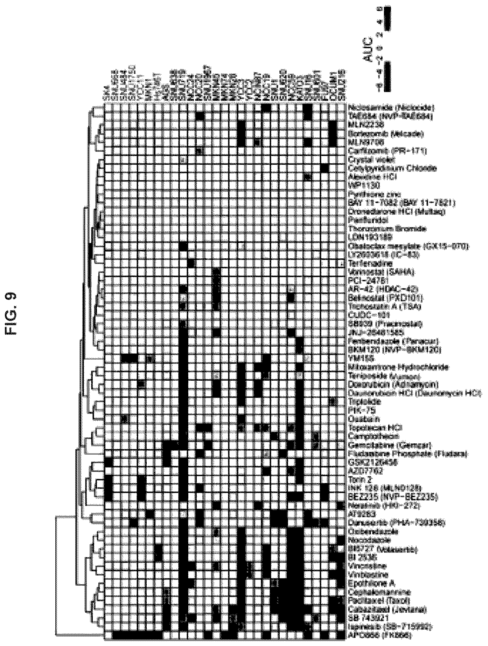

Cells cultured for cell-based drug assay were inoculated into a 96-well white optical plate (Corning) at 5 × 10³ cells per well. After 24 hours after inoculation, a 2.5 MJT (primary screen) or a half-log 12-serial diluent (secondary screen) of the pharmacological compound contained in DMSO is added to the cells with a robot using a BioMek FXp liquid processor (Beckman) to add to the cells at 50 mM. The final drug concentration in the 5 ns range and the DMSO specification control (0.5%) were calculated. The cells were then further incubated at 37°C in a 5% CO incubator for 72 hours prior to measuring cell viability using the extra-GLO assay kit (Promega). The luminescent properties were measured after 15 min incubation at room temperature using a plate stacker equipped with a plate stacker. The normalized viability for the DMSO-based control was measured to generate a cell line-specific 12-point dose-response curve for each pharmacological compound, and then the area under the curve (AUC) was calculated using a trapezoidal method. The average area under the viability curve (AUC) is a value that exhibits high cell line sensitivity to drugs. The results of the repeated experiments were averaged to generate AUC values for each cell line for each compound. The results are shown in FIG. 9 and Tables 4 to 12.

**TABLE 4**

| | **AZD7762** | **Cabazitaxel (Jevtana)** | **Danusertib (PHA-739358)** | **INK 128 (MLN0128)** | **MLN9708** | **PCI-24781** | **TAE684 (NVP-TAE684)** |
|---|---|---|---|---|---|---|---|
| **SK4** | -0.96959 | -0.72984 | -0.66774 | -2.93099 | 0.40776 | 0.45102 | -0.65724 |
| **SNU668** | 1.53671 | 0.4109 | 1.04925 | 1 .08497 | 1 .23248 | 0.52999 | 1.00892 |
| **SNU484** | 0 | -0.72784 | -1.53715 | -1 .08939 | -1.06877 | 0.15333 | -0.61817 |
| **SNU1750** | -0.9031 | 0.60578 | -2.3091 | -1.07908 | -2.16067 | -1.49924 | -0.94102 |
| **YCC11** | -0.97566 | -1.18917 | -1.35668 | -2.64831 | -1.82448 | -0.09531 | -1.4836 |
| **MKN1** | -0.01906 | -1.26587 | -0.8271 | -0.74437 | 0.06734 | 0.65806 | 0 |
| **Hs746T** | 1.37623 | 1.08466 | 1.56452 | -1 .82054 | -1.54738 | -0.03183 | 1.03945 |
| **AGS** | -0.44033 | -2.20138 | -1.03361 | -1.24411 | -0.69884 | -1.16146 | -1 .32629 |
| **SNU638** | 1.40403 | -0.1608 | -0.21452 | 0.23868 | 0.0059 | -0.06055 | 0.40708 |
| **SNU719** | 3.77086 | 6.20873 | 3.93493 | 3.8255 | 1 .30434 | 1.42811 | 1.05145 |
| **NCC24** | 1.10699 | -1.07721 | 0.98786 | 0.75018 | -0.90855 | -0.27657 | 1.66186 |
| **NCC20** | -0.41669 | -0.85509 | -4.08194 | -2.56833 | -1 .52705 | -1.06517 | -2.65881 |
| **SNU1967** | -0.36261 | 1.22763 | 0.03541 | -0.47275 | -1 .55646 | -1.12621 | -1.41193 |
| **MKN45** | -0.3384 | 2.13796 | 2.10321 | -0.59353 | 0.25811 | 2.25891 | 2.60112 |
| **MKN74** | -0.47726 | 0.43188 | 0.13208 | -0.48826 | -0.40082 | 0.21277 | -0.04126 |
| **MKN28** | -0.31308 | -2.21296 | 1 .24506 | 1 .19513 | 0.1368 | -0.37804 | -0.41772 |
| **YCC3** | 1.63754 | 3.38425 | 0.79491 | 0.85345 | 2.71673 | 0.84261 | 1.70841 |
| **YCC2** | 0.45099 | 0.20727 | -0.99006 | 0 | 0.33774 | 0.00064 | -0.55122 |
| **NCI N87** | 0.84046 | -1.71241 | 2.12232 | 0.70815 | 1.9321 | -0.38027 | 2.00312 |
| **NCC19** | 2.79573 | 0 | 1.88453 | 0.30218 | -0.79134 | 0.32816 | 1 .94362 |
| **SNU1** | -0.20255 | -3.45074 | -2.27449 | 1.28142 | 0.02493 | 0.34291 | -0.11849 |
| **SNU620** | 0.22575 | -3.50061 | -2.61551 | 0.08832 | -0.70355 | -0.69661 | 0.46365 |
| **NCC59** | 2.10683 | 3.93854 | 1.63173 | 1.54134 | -0.34722 | 0.97658 | 1.27316 |
| **KATO3** | 2.89068 | 4.94965 | -0.30456 | 1.63301 | 0.55904 | -0.21112 | 0.26725 |
| **SNU16** | -1.11884 | -2.75865 | -3.76037 | -0.87335 | -2.25873 | -1 .23299 | -3.18034 |
| **SNU601** | -0.95704 | -1.68818 | 2.87723 | 1.34567 | 0 | 0.61406 | 1.06167 |
| **FU97** | -0.7616 | 0.30606 | -2.48769 | -2.85716 | -1.56756 | -1.78622 | -1.58701 |
| **OCUM1** | 0.51097 | 3.08628 | 0.87419 | 0.03105 | 2.09708 | 0 | -0.21947 |
| **SNU216** | 0.70035 | 2.33498 | 0 | -0.90819 | 0.14158 | 0.03042 | 0.52052 |

**TABLE 5**

| | **BAY 11-7082 (BAY 11-7821)** | **Camptothecin** | **Daunorubicin HCl (Daunomycin HCl)** | **Fludarabine Phosphate (Fludara)** | **lspinesib (SB-715992)** | **Neratinib (HKI-272)** | **Penfluridol** |
|---|---|---|---|---|---|---|---|
| **SK4** | -0.76588 | -0.88232 | 0 | -1.65271 | -0.82668 | -1.28205 | -0.50755 |
| **SNU668** | 0.8081 | -1.20186 | -0.91418 | -1.51064 | 0.10008 | 0.8456 | 0.00275 |
| **SNU484** | -0.45038 | 1.04736 | -1 .29932 | 1.30916 | -0.95095 | -1.2895 | -0.39575 |
| **SNU1750** | -1.01204 | 0.02348 | -0.77953 | 1.97191 | 0.22654 | -0.41235 | -0.03209 |
| **YCC11** | -1.53808 | -0.57179 | -1.74218 | -0.64169 | -1.38089 | -1.33339 | -0.51645 |
| **MKN1** | -0.32482 | -0.64196 | -0.28772 | 0.97476 | -1.17815 | -0.49849 | -0.01646 |
| **Hs746T** | -0.5796 | 0.3026 | 0.14844 | 1.97034 | 1.04625 | -0.22041 | 0.28215 |
| **AGS** | -0.5085 | -1 .98865 | -1.2278 | -2.78252 | -2.13137 | -0.93145 | -0.69168 |
| **SNU638** | -1.49699 | -0.93655 | -1 .22938 | 0.50061 | -0.20302 | 0 | -1 .32035 |
| **SNU719** | 1.37741 | 3.07877 | 3.41631 | 2.04643 | 2.54219 | 1.89357 | 0.3303 |
| **NCC24** | 0.83571 | 0.52733 | -0.88006 | 1.03684 | -0.83483 | 1.20488 | 0.50232 |
| **NCC20** | -0.82871 | -1.82853 | -0.77365 | -2.50704 | -0.99388 | -1.6785 | -0.84708 |
| **SNU1967** | -0.80276 | -1.85951 | -1.48144 | 2.18583 | 1.56163 | 0.83812 | -0.08879 |
| **MKN45** | 0.19396 | 0.12032 | 2.94926 | -1.02931 | 1.83317 | 3.02852 | 0.9981 |
| **MKN74** | 0.03633 | -0.08807 | 1 .21514 | -0.17305 | 0 | -1.14443 | -1.13276 |
| **MKN28** | -0.19868 | 0.38815 | 2.14551 | 0.00328 | -1.95588 | 0.18644 | -0.60969 |
| **YCC3** | 0.71162 | 1.3493 | 2.69895 | 0 | 2.00727 | 0.30133 | 0.4477 |
| **YCC2** | 0 | 0 | 1 .12717 | -0.7765 | 0.79868 | -0.17485 | -0.07658 |
| **NCI N87** | 0.50726 | 0.72611 | 4.5372 | -0.06169 | -0.0052 | -5.05094 | 0.3682 |
| **NCC19** | -0.07527 | 0.98541 | 0.93573 | 2.41081 | 1.11079 | 2.05245 | 0.62388 |
| **SNU1** | 1.33024 | -2.18172 | -0.76053 | -0.76442 | -1.68 | 1.03788 | 0.84791 |
| **SNU620** | -0.19225 | -1 .86656 | -1 .23899 | -2.28897 | -1 .25459 | 1.11678 | 0.04726 |
| **NCC59** | 1.03064 | 1.20816 | -0.51608 | 1.47865 | 2.6543 | 1.9785 | 0.34391 |
| **KATO3** | 0.72294 | 4.76878 | 6.28841 | 1.16487 | 2.99855 | 1.50538 | 0 |
| **SNU16** | -0.67151 | -1.52878 | -1 .06545 | -0.43997 | -1.93529 | -1.491 | -1.2587 |
| **SNU601** | 0.72182 | -1.85259 | 1.20701 | -0.5439 | -1.37063 | 0.80204 | 0.67479 |
| **FU97** | 0.0931 | 1.09653 | 0.39653 | 2.92809 | 1.3405 | -0.2016 | 0.09295 |
| **OCUM1** | 0.74911 | -0.50334 | 0.70658 | -0.39673 | 1 .29694 | 1.05627 | 0.2066 |
| **SNU216** | 0.38649 | 1.3094 | 0.45068 | 1.77139 | 1.80082 | -2.1011 | -0.50693 |

**TABLE 6**

| | **Teniposide (Vumon)** | **Vinblastine** | **Alexidine HCl** | **Belinostat (PXD101)** | **Carfilzomib (PR-171)** | **Doxorubicin (Adriamycin)** | **JNJ-26481585** |
|---|---|---|---|---|---|---|---|
| **SK4** | -0.2681 | -1.0813 | -0.90233 | -0.3048 | -0.24149 | 0.21297 | -0.32583 |
| **SNU668** | -0.61746 | -1.03703 | -0.13749 | 0.69077 | 1 .89863 | -0.39086 | 1 .48608 |
| **SNU484** | -1.21094 | -1.19408 | -1.21174 | 0.99658 | -0.76023 | -0.84178 | 0 |
| **SNU1750** | -0.56177 | 0.64969 | -0.63305 | -0.90196 | -0.9787 | -0.95764 | -0.85964 |
| **YCC11** | -1.34153 | -0.61594 | -1.34704 | -0.55558 | -0.76069 | -1.87689 | 0.08841 |
| **MKN1** | -1.95464 | -0.9285 | 0.86874 | 0.9981 | 0.17712 | -0.20162 | 0.55802 |
| **Hs746T** | 0.62607 | 1.1779 | -0.25966 | 0.55979 | -1 .39924 | -0.08449 | 0.29058 |
| **AGS** | -2.04166 | -1 .12929 | -0.44398 | -0.93715 | -0.13102 | -1.08077 | -1 .07273 |
| **SNU638** | -0.35845 | 0.26525 | -0.61203 | -0.50115 | 0.14636 | -0.33421 | 0.1995 |
| **SNU719** | 2.89859 | 4.16203 | 1.19955 | 2.32905 | 0.51783 | 4.66047 | 3.52667 |
| **NCC24** | -0.57665 | -0.9438 | -0.47448 | -1.09498 | -0.8995 | -0.61259 | -1 .59203 |
| **NCC20** | -0.87155 | -1.61429 | 0.69634 | 0 | -2.00228 | -0.48795 | -1.39619 |
| **SNU1967** | -0.7519 | -0.94114 | 0.18277 | -0.31218 | -0.55865 | -0.87867 | -1.0365 |
| **MKN45** | 1.93269 | 1.84439 | 0.04553 | 2.49342 | 0.55529 | 2.50486 | 2.74332 |
| **MKN74** | 0.39926 | 0.66387 | -0.34328 | -0.18352 | -0.31622 | 1.52242 | -0.06428 |
| **MKN28** | 0.10085 | -0.818 | 0 | -0.39525 | -0.70651 | 2.97966 | -0.53115 |
| **YCC3** | 2.1949 | 4.54047 | 0.82726 | 1.06489 | 1.74674 | 2.8776 | 0.35609 |
| **YCC2** | 0.24899 | 2.05959 | -0.00823 | 0.17063 | 0.85339 | 1.4121 | -0.10178 |
| **NCI N87** | 2.31934 | -0.17212 | 2.53075 | 0.02343 | 0.50977 | 6.89113 | -0.26494 |
| **NCC19** | 2.41523 | 2.90508 | 1.13183 | 1.20567 | 1.56176 | 2.86662 | 0.39122 |
| **SNU1** | -0.30191 | -0.39488 | 0.70611 | 0.11466 | 0.13905 | 0 | -0.98405 |
| **SNU620** | 0 | -0.49087 | 0.0423 | -1.0681 | -0.42418 | -0.20094 | -1.2467 |
| **NCC59** | 1.58987 | 2.83336 | 1.33415 | 2.68319 | 0.85921 | 1.23071 | 3.68716 |
| **KATO3** | 1.85247 | 4.09597 | 0.541 | 0.75442 | 0.10045 | 7.9338 | 0.34816 |
| **SNU16** | -0.81923 | -1.30003 | -1.84911 | -1.57712 | -1.3183 | -0.0398 | -0.78834 |
| **SNU601** | -1.09439 | 0 | 1.89809 | 1.49489 | 1 .48888 | 4.48116 | 1.50128 |
| **FU97** | 1.30852 | 0.37717 | -0.9535 | -0.97262 | -0.16869 | -1.23746 | -0.63245 |
| **OCUM1** | 1.00066 | 2.51438 | -0.04134 | -0.22495 | 1 .24478 | 0.67879 | 0.02955 |
| **SNU216** | 0.92958 | 2.53865 | 0.03777 | -0.2541 | 0 | 0.74663 | 0.34242 |

**TABLE 7**

| | **Niclosamide (Niclocide)** | **Terfenadine** | **Vincristine** | **APO866 (FK866)** | **BEZ235 (NVP-BEZ235)** | **Cephalomannine** | **Dronedarone HCl (Multaq)** |
|---|---|---|---|---|---|---|---|
| **SK4** | -1.03768 | -0.87128 | -1.28286 | 0.74033 | -3.14195 | -1.14434 | -0.34878 |
| **SNU668** | -0.90838 | -0.56472 | -1.31484 | -3.30444 | 1.25801 | -0.19115 | 0.41227 |
| **SNU484** | -1.16377 | 0.06057 | -1.96535 | -6.82326 | 0 | -0.7051 | -0.24967 |
| **SNU1750** | -0.89874 | -0.05758 | 0 | -3.76719 | -1.12874 | 1.00866 | 0.06657 |
| **YCC11** | -0.87526 | -0.99605 | -1.41982 | -2.21518 | -2.69815 | -1.01251 | -0.52095 |
| **MKN1** | 0.39914 | -0.81275 | -1.53038 | -5.50199 | -0.84497 | -1.48574 | 0 |
| **Hs746T** | -0.68585 | -0.32462 | 1.04359 | -4.38171 | -0.9926 | 0.44895 | -0.03562 |
| **AGS** | -1.09355 | -1.27816 | -1.75048 | 0 | -1.76804 | -2.05472 | -0.64154 |
| **SNU638** | -1.10519 | -0.80908 | 0.13479 | 0.60324 | 0.89791 | -0.03958 | -0.56488 |
| **SNU719** | 2.50346 | 1.05885 | 5.03615 | 1.27532 | 5.11866 | 4.89284 | 1.29094 |
| **NCC24** | 0.67997 | -2.35459 | -3.15623 | -0.07585 | 0.08486 | -0.77162 | 1.04449 |
| **NCC20** | 0.21767 | -1.02876 | -1.43374 | 2.34977 | -1 .54232 | -0.50109 | -0.29847 |
| **SNU1967** | 0.14186 | -1.06535 | -1.3653 | -3.65274 | -0.16999 | 0.65249 | 0.19188 |
| **MKN45** | 1.15938 | -0.54412 | 2.22568 | 0.61912 | 1 .88682 | 0 | 0.67723 |
| **MKN74** | -1.62004 | -1.58864 | 0.209 | -6.52962 | 0.02041 | 0.35277 | -1.38074 |
| **MKN28** | -0.38089 | -0.29012 | -0.49305 | -6.85466 | 2.00741 | -1.69739 | -0.1927 |
| **YCC3** | 1.8253 | 1.42339 | 4.6576 | 1 .42262 | 0.90084 | 2.80935 | 0.88162 |
| **YCC2** | 0.66277 | 0.48617 | 2.29757 | 1.33071 | 0.64029 | 0.38382 | -0.08246 |
| **NCI N87** | 2.80165 | 1.46517 | 1.61472 | 0.83429 | 0.75054 | -1.25982 | -0.08952 |
| **NCC19** | 2.91749 | 3.13466 | 5.03916 | -4.01049 | 0.90219 | 1.7094 | -0.37107 |
| **SNU1** | -1.67279 | 1.33992 | -0.5253 | 0.97263 | -1.12835 | -2.05619 | 1.29453 |
| **SNU620** | 0.85178 | 0.49182 | -0.37923 | 0.34879 | -0.68933 | -2.40264 | -0.33527 |
| **NCC59** | 1.14057 | 2.3563 | 3.84746 | 0.79188 | 2.99705 | 2.73396 | 0.67691 |
| **KATO3** | 1.59054 | 0.49008 | 4.8981 | 1.55328 | 2.71695 | 4.17856 | 0.17208 |
| **SNU16** | -3.06247 | 0 | -1.52206 | -4.56166 | -0.98587 | -1.73683 | -0.21488 |
| **SNU601** | 4.91115 | 2.64114 | 0.93811 | 4.97639 | 2.66043 | 0.2225 | 3.84768 |
| **FU97** | -1.79266 | 1 .16326 | -1.11525 | -3.14919 | -2.38238 | 0.41071 | 0.38071 |
| **OCUM1** | 0 | 1.03176 | 0.65483 | 0.94898 | -0.37194 | 1.3887 | 0.02571 |
| **SNU216** | -0.51854 | 1.49883 | 1.75519 | -5.89656 | -0.63506 | 1.8612 | 0.08211 |

**TABLE 8**

| | **Nocodazole** | **PIK-75** | **Thonzonium Bromide** | **Vorinostat (SAHA)** | **AR-42 (HDAC-42)** | **BI 2536** | **Cetylpyridinium Chloride** |
|---|---|---|---|---|---|---|---|
| **SK4** | -0.37398 | 1.00916 | -1.03277 | 0.64343 | -0.36166 | 1.12752 | -1.31206 |
| **SNU668** | -0.1188 | -0.42799 | 0.31512 | 0.43033 | 0.25146 | -0.42108 | -0.02952 |
| **SNU484** | -0.81362 | 0.94692 | -0.90815 | 1.04054 | 0.12698 | 0 | -0.74014 |
| **SNU1750** | 0.75023 | 0 | 0.06729 | -0.57367 | -1.40057 | 0.80762 | -0.36132 |
| **YCC11** | -0.82792 | -0.5398 | -0.8304 | -0.22846 | -0.3298 | 0.38175 | -1.12912 |
| **MKN1** | -0.65187 | 0.03458 | -0.24138 | 0.42818 | 0.9532 | 0.21226 | 0.99937 |
| **Hs746T** | 1.27996 | 0.1171 | 0 | -0.04774 | 0.3259 | 1 .95082 | 0.2943 |
| **AGS** | -1.57279 | -1.2576 | -0.41051 | -0.59466 | -1.18563 | 1.21971 | -0.10439 |
| **SNU638** | -0.18754 | 1.31203 | -0.4973 | -0.43133 | -0.78036 | 0.18629 | -0.31422 |
| **SNU719** | 4.97609 | 5.72582 | 1.90015 | 1.84324 | 2.16929 | 3.29061 | 1.71819 |
| **NCC24** | 0 | 0.07825 | -0.24645 | -0.34214 | -0.62174 | 0.88322 | 0.06564 |
| **NCC20** | -1.69461 | 0.49134 | -0.60416 | -0.93283 | 0.06736 | 0.57219 | -0.23178 |
| **SNU1967** | -0.07292 | 0.30481 | 0.01501 | -0.25741 | -0.91371 | 0.0237 | -0.50013 |
| **MKN45** | 2.00497 | 1 .16958 | 0.12127 | 2.41555 | 1.96173 | 0.09661 | 0 |
| **MKN74** | 0.70808 | 0.20524 | -0.99665 | -0.07666 | 0.17888 | -0.6584 | -0.41764 |
| **MKN28** | -0.14781 | 0.05865 | 0.07021 | -0.08142 | 0.07777 | 1.69041 | 0.48765 |
| **YCC3** | 3.06603 | 1.72349 | 0.61631 | 1.03355 | 0.68754 | 3.31495 | 0.39005 |
| **YCC2** | 1.3363 | 1 .28946 | -0.42546 | 0.09328 | -0.00569 | 0.18755 | 0.14211 |
| **NCI_N87** | -0.78432 | 0.12381 | 0.41496 | 0.89861 | -0.13445 | 1.00571 | 1.07471 |
| **NCC19** | 0.87171 | 0.35347 | 0.29949 | 0.2993 | 1.02553 | 3.47544 | 1 .20462 |
| **SNU1** | -0.72421 | 0.33973 | 1.47807 | -0.21515 | -0.31471 | 1.06385 | 0.51714 |
| **SNU620** | -0.73499 | 0.78819 | -0.24037 | 0.08594 | -1.19727 | 1.07787 | 0.09525 |
| **NCC59** | 3.0345 | 0.15646 | 0.60964 | 1.00996 | 2.10183 | 3.55559 | 1.49737 |
| **KATO3** | 3.18806 | 3.79267 | -0.12702 | 0 | 0.73241 | 4.70426 | 1.07164 |
| **SNU16** | -1.50716 | 1.48525 | -0.91283 | -0.78753 | -1.42669 | 0.02031 | -0.15341 |
| **SNU601** | 1.74275 | 1 .99904 | 2.22763 | 2.84015 | 1.87435 | 0.0215 | 2.32847 |
| **FU97** | 0.37972 | 0.52672 | -0.03008 | 0.29755 | -1.35793 | -0.4841 | -2.03913 |
| **OCUM1** | 0.72477 | 0.51054 | 0.22087 | -0.51657 | -0.39363 | 2.22645 | -0.14407 |
| **SNU216** | 2.03227 | 0.8132 | 0.27372 | -0.04999 | 0 | 2.03806 | -0.14827 |

**TABLE 9**

| | **Gemcitabine (Gemzar)** | **LDN193189** | **Obatoclax mesylate (GX15-070)** | **Topotecan HCl** | **WP1130** | **AT9283** | **BI6727 (Volasertib)** |
|---|---|---|---|---|---|---|---|
| SK4 | -0.87645 | -0.53392 | -1 .53083 | -0.45152 | 0.08364 | -0.4441 | -0.92628 |
| SNU668 | -1.31169 | 0.06394 | -0.27152 | -1.12816 | 0.16066 | 0.69683 | 0.15164 |
| SNU484 | -0.27565 | -0.59991 | -1.40038 | 1.12806 | 0.89708 | 1.87241 | -0.03282 |
| **SNU1750** | 0 | -0.46954 | -0.87205 | 0.28185 | 0.86017 | -1.7502 | -0.59616 |
| **YCC11** | -1.64941 | -0.73587 | -0.84068 | -0.57299 | 0.21436 | 1.46744 | -0.79966 |
| **MKN1** | -0.91691 | 0 | 0.78568 | -0.99541 | 0.42644 | -2.315 | -0.16202 |
| **Hs746T** | 1.70982 | -0.29542 | 0 | 0.45868 | 0.37378 | 0.9084 | 2.37838 |
| **AGS** | -2.36417 | -0.18957 | -0.90049 | -2.15921 | 0.69975 | 0.93026 | -1.0975 |
| **SNU638** | -2.40365 | -0.96995 | -0.50945 | -0.71325 | 0.34846 | 0.01142 | 0.36259 |
| **SNU719** | 3.62772 | 0.60431 | 2.58471 | 3.06861 | 1.24502 | 3.01867 | 3.74048 |
| **NCC24** | -1.10009 | -0.14638 | 0.25109 | 0.0033 | 0.34748 | -0.6909 | -0.95023 |
| **NCC20** | -0.90889 | 0.02059 | -0.98675 | -2.36704 | 0.82472 | 2.94101 | 0.55942 |
| **SNU1967** | -4.70451 | -1.41897 | -0.40934 | -2.64781 | 0.71953 | 0.12444 | -0.16446 |
| **MKN45** | 1.05269 | 0.95163 | 0.93127 | 1.19453 | 1.02413 | 0.19963 | 0.58753 |
| **MKN74** | -1.40168 | -1.24585 | -1.05119 | 0 | 0.19079 | 0.4728 | -0.01134 |
| **MKN28** | 1.08904 | 0.57577 | -0.37689 | 1.25921 | 0.04006 | 1 .90297 | -1.33459 |
| **YCC3** | 0.18526 | 0.08342 | 2.28079 | 1.92331 | 0.87839 | 0.66186 | 3.48761 |
| **YCC2** | 0.11113 | 0.11632 | -0.59155 | 0.1906 | 0.94002 | 0.07527 | -0.09826 |
| **NCI N87** | 1.32966 | 1.17156 | 0.63597 | 2.85714 | 2.15893 | 0.1418 | -0.39292 |
| **NCC19** | 1.69737 | 0.06094 | 1.60427 | 2.89621 | 1.37612 | 2.24675 | 3.25648 |
| **SNU1** | -0.33667 | 0.87058 | 0.52379 | -0.61631 | 1.52716 | 3.74346 | -0.48702 |
| **SNU620** | -1.63964 | -2.13071 | 0.81287 | -0.76112 | 1.33608 | 4.05927 | -0.40977 |
| **NCC59** | 3.02402 | 1.89232 | 2.5912 | 1.95697 | 0.75884 | 0.58817 | 3.19822 |
| **KATO3** | 4.1466 | 0.62048 | 1 .38324 | 5.01014 | 1 .45416 | 0.5115 | 3.3921 |
| **SNU16** | 0.51403 | 1.52496 | 1 .84075 | -0.52924 | 0 | 3.80867 | 0 |
| **SNU601** | 0.24712 | 3.69936 | 2.79142 | -0.55889 | 3.23969 | 1.64753 | 0.47568 |
| **FU97** | 1.88834 | -0.14339 | -1.5029 | -0.84454 | -0.7929 | 0.19243 | 0.30776 |
| **OCUM1** | -1.40169 | -1.11321 | 0.13024 | -1 .68063 | 0.65197 | 0 | 2.40542 |
| **SNU216** | 1.83738 | -0.78382 | -0.13978 | 1.12337 | 0.11013 | 0.53227 | 1 .86845 |

**TABLE 10**

| **A** | **Epothilone A** | **LY2603618 (IC-83)** | **Ouabain** | **Pyrithione zinc** | **Torin 2** | **YM155** | **BKM120 (NVP-BKM120)** |
|---|---|---|---|---|---|---|---|
| **SK4** | -0.95406 | -1.69816 | -1.41445 | 0 | 2.72175 | 1.31438 | -1.24718 |
| **SNU668** | -0.29093 | 0.17133 | -1.02595 | 0.76814 | 0.43911 | -0.2961 | 0.26885 |
| **SNU484** | -0.43639 | 0.31413 | -2.02983 | -0.92867 | -0.505 | 2.55976 | -0.47501 |
| **SNU1750** | 1 .03899 | -0.4819 | -0.73744 | 0.10112 | 0.78704 | 2.62448 | -0.43924 |
| **YCC11** | -1.2702 | -2.02695 | -0.34942 | -1.33727 | 2.29864 | 1.41945 | -1.14691 |
| **MKN1** | -1.13987 | -1 .02259 | -1.22288 | -0.49131 | 1.57382 | -2.0115 | -0.18271 |
| **Hs746T** | 1.21714 | -0.10702 | 0.30933 | -0.54101 | 0.75813 | 1.37593 | 0 |
| **AGS** | -1.65509 | -0.3593 | -0.43512 | -0.93555 | 1.70136 | 1.51172 | -1.09389 |
| **SNU638** | 0.31182 | -0.05048 | 1.054 | -0.08685 | 0.3739 | 0.50985 | 0.01503 |
| **SNU719** | 4.48401 | 2.40356 | 5.03822 | 1.54675 | 3.84089 | 3.78941 | 3.31384 |
| **NCC24** | -1.8431 | -1.44478 | -0.24166 | -0.30898 | 0 | -0.6903 | -0.43906 |
| **NCC20** | -0.54593 | 0.01673 | 0.23646 | -0.78379 | 1.59762 | 0 | -0.07502 |
| **SNU1967** | 0.64772 | -0.96158 | 0.98734 | -0.50269 | 0.55703 | 0.20436 | 0.67599 |
| **MKN45** | 0.66045 | -0.94724 | -0.36649 | -1.0871 | 0.67038 | 0.67918 | 1 .07422 |
| **MKN74** | 0.29134 | -0.90838 | -0.94721 | -1.14856 | 0.18682 | 1.30495 | -0.05182 |
| **MKN28** | -1.4393 | -0.1902 | -0.51826 | 0.14213 | 0.8861 | 1.02102 | -0.0925 |
| **YCC3** | 2.8742 | 0.23046 | 4.20868 | 0.65119 | 1.39941 | 1.32464 | 1.08466 |
| **YCC2** | 0.17839 | 0.04045 | -0.67849 | -0.08387 | 0.06527 | 0.42281 | 0.71694 |
| **NCI N87** | -1.50992 | 0.87711 | 0.91918 | 1.16121 | 0.30783 | 0.41677 | -0.38005 |
| **NCC19** | 0 | 1.2209 | 0.38743 | 0.73363 | 1.61395 | 3.60768 | 0.68658 |
| **SNU1** | -1.74991 | -0.33995 | -0.4741 | 1.43099 | 1.39165 | 1.21155 | 0.25564 |
| **SNU620** | -2.26821 | 0 | 0 | -0.78173 | 0.39651 | 1.29004 | -0.08823 |
| **NCC59** | 3.58289 | 0.93261 | 1.56721 | 0.72882 | 1.58432 | 0.13858 | 1 .23031 |
| **KATO3** | 4.76147 | 1 .25634 | 4.78341 | 0.82189 | 2.65978 | 0.30807 | 2.44864 |
| **SNU16** | -1.12023 | 1.46675 | 2.22926 | 1 .28045 | 0.9803 | 2.49675 | -0.30757 |
| **SNU601** | -0.20062 | 0.2367 | 2.70192 | 2.66622 | 0.41036 | 1 .66053 | 0.97107 |
| **FU97** | 0.94544 | 0.71107 | -0.38887 | 0.33441 | 1.52612 | 1.30071 | -1.3007 |
| **OCUM1** | 1.69909 | -0.68305 | 0.11126 | 0.53552 | 0.11271 | 1.68528 | 0.75609 |
| **SNU216** | 2.40328 | 0.24621 | 0.20093 | -0.22721 | 0.49637 | 0.09958 | 0.13416 |

**TABLE 11**

| violet | **Crystal violet** | **Fenbendazole (Panacur)** | **GSK2126458** | **Mitoxantrone Hydrochloride** | **Oxibendazole** | **SB 743921** | **Trichostatin A (TSA)** |
|---|---|---|---|---|---|---|---|
| **SK4** | -1.01459 | -0.29532 | -2.46578 | -0.61266 | -0.14145 | 0.97693 | 0.78971 |
| **SNU668** | 0.19369 | 0 | 1.58711 | -0.65807 | -0.36382 | 0 | 0.13439 |
| **SNU484** | -0.71083 | -0.08111 | 0.46406 | -1.15828 | -0.00299 | -1.3076 | 0.45152 |
| **SNU1750** | -0.55145 | 0.25963 | -0.50708 | -0.35869 | 0.4952 | 0.00014 | -1.0771 |
| **YCC11** | -0.89961 | -0.73051 | -1.37286 | -1.25152 | -0.29015 | 1.35302 | -0.26947 |
| **MKN1** | 0.42106 | -0.0568 | -1.1278 | -1.19659 | -0.58595 | 1.40636 | 0.42233 |
| **Hs746T** | -0.20848 | 0.57281 | -0.063 | 0.59247 | 1 .20383 | 1 .35856 | 0.66212 |
| **AGS** | -0.47692 | -1.15953 | -2.28613 | -1.67098 | -1.06391 | -2.36598 | -1.13749 |
| **SNU638** | -0.49565 | -0.36892 | 0.9531 | -1.05993 | -0.28076 | 0.25718 | -0.27578 |
| **SNU719** | 3.07583 | 3.38225 | 5.21129 | 3.64711 | 3.17002 | 2.11498 | 1.88329 |
| **NCC24** | 0.27433 | -0.84877 | -1.19745 | -1.03812 | -1.131 | 2.23809 | -1.30687 |
| **NCC20** | -1.03062 | -0.5211 | -1.10669 | -0.74225 | -0.74664 | 1.28847 | -1.84165 |
| **SNU1967** | 0.34585 | 0.18401 | 1.04368 | -1.34228 | 0 | 0.85722 | -1.47245 |
| **MKN45** | 1.10103 | 1.40735 | 0.05353 | 2.014 | 2.18686 | 2.81808 | 2.21517 |
| **MKN74** | -0.57212 | -0.28025 | 0 | -0.29145 | 0.44981 | 0.00599 | 0.30048 |
| **MKN28** | 0 | -0.78769 | 0.15004 | 1.61564 | -0.13066 | 1.69905 | -0.65155 |
| **YCC3** | 0.94374 | 1.37604 | 0.97454 | 2.27028 | 2.89651 | 2.42138 | 1.16995 |
| **YCC2** | 0.42893 | 0.56953 | -0.92593 | 0.51257 | 0.7894 | 0.20477 | 0.20173 |
| **NCI_N87** | 0.49073 | -1.12375 | -1.3463 | 2.6755 | -0.19275 | 1.20548 | -0.01232 |
| **NCC19** | 0.48728 | -0.27791 | 0.46653 | 2.88363 | 1.21934 | 1.29576 | -0.30257 |
| **SNU1** | -0.53549 | 0.16291 | -0.64916 | 0 | -1.00691 | -0.9587 | -0.99459 |
| **SNU620** | -0.76722 | -1.425 | -0.79455 | -0.60117 | -1.52778 | 2.36575 | -1.34254 |
| **NCC59** | 0.50975 | 1.9513 | 2.2975 | 1 .00074 | 2.51829 | 2.28499 | 1.78447 |
| **KATO3** | 0.91103 | 2.34414 | 3.91116 | 4.80703 | 3.47172 | 3.18865 | -0.15551 |
| **SNU16** | -0.56685 | 1.2297 | 0.64936 | 1 .60005 | 2.326 | 1.45395 | 1.14131 |
| **SNU601** | 0.94416 | 0.59899 | 0.10472 | -0.52507 | 1 .09509 | 1.70304 | 1.15459 |
| **FU97** | -0.98502 | -1.85427 | -1 .39298 | 0.4542 | -0.35477 | 0.84069 | -1.18131 |
| **OCUM1** | 0.77411 | 1.28281 | 1.22686 | 0.80785 | 1.05852 | 1 .65279 | 0.14745 |
| **SNU216** | -0.02818 | 1.005 | -0.47751 | 1.1656 | 1.74368 | 2.43774 | 0 |

**TABLE 12**

| | **Bortezomib (Velcade)** | **CUDC-101** | **MLN2238** | **Paclitaxel (Taxol)** | **SB939 (Pracinostat)** | **Triptolide** |
|---|---|---|---|---|---|---|
| SK4 | 0.39441 | -0.04342 | 0.5584 | -0.87202 | -0.45404 | -1.23208 |
| SNU668 | 1.35806 | 0.95079 | 1.1951 | -0.02282 | 0.51901 | -1.28431 |
| SNU484 | -0.63378 | 0.58306 | -0.41249 | -0.36037 | 0.208 | -1.23432 |
| SNU1750 | -1.32572 | -1.20297 | -1.89594 | 0.81096 | -1.15638 | -0.42134 |
| YCC11 | -0.66493 | 0.34513 | -1.69454 | -0.83022 | -0.768 | 0.41443 |
| **MKN1** | 0.3149 | 0.62674 | 0.39585 | -1.25779 | 0.23196 | 0.67716 |
| **Hs746T** | -0.9394 | 0.4609 | -1.18892 | 0.73422 | 0.70977 | 0.16997 |
| **AGS** | -0.1894 | -0.95687 | -0.80119 | -1.83091 | -1.79948 | -1.26743 |
| **SNU638** | 0.06564 | -0.88065 | -0.57363 | -0.38984 | -0.27801 | 0.82796 |
| **SNU719** | 1.08149 | 0.93484 | 0.41685 | 4.87652 | 2.18718 | 4.57324 |
| **NCC24** | -0.39113 | 0.18137 | -1.21033 | -1.61582 | -1.13695 | -1.65601 |
| **NCC20** | -0.10784 | -0.78848 | -1.24668 | 0 | -0.08068 | 0 |
| **SNU1967** | -0.84527 | -0.30099 | -1.67628 | 0.05016 | -0.45813 | -0.49017 |
| **MKN45** | 0.16409 | 1.50017 | 0.40055 | 1.47343 | 1.67243 | 1 .15654 |
| **MKN74** | 0 | -0.54066 | -0.22141 | 0.18599 | 0.38059 | -0.82311 |
| **MKN28** | -0.41601 | -0.76633 | 0.52567 | -0.27308 | -0.58656 | -0.03132 |
| **YCC3** | 2.79051 | 0.75618 | 2.80758 | 3.80989 | 1.40282 | 2.61978 |
| **YCC2** | 0.69533 | -0.1991 | 0.04339 | 0.56726 | -0.02045 | 0.63092 |
| **NCI N87** | 1.56918 | -0.42172 | 1.70782 | -1.01263 | -0.67244 | -0.78676 |
| **NCC19** | -0.69688 | 1.18709 | -0.81661 | 1.90196 | 0.65384 | -1.50739 |
| **SNU1** | -0.30281 | 0 | -0.5037 | -1.77813 | -1.39979 | -0.12074 |
| **SNU620** | -0.6115 | -0.42889 | -0.96426 | -1.99824 | -1.19909 | -0.98604 |
| **NCC59** | 0.8821 | 1.8155 | 0.31796 | 3.63654 | 1.05279 | 0.35598 |
| **KATO3** | 1.08177 | 0.68296 | 0.71149 | 5.3572 | 0.21731 | 5.71479 |
| **SNU16** | -0.0987 | 0.42696 | 0.77009 | -0.51047 | -1.06697 | 1.9544 |
| **SNU601** | -0.07383 | -0.1084 | -0.2758 | -1.10598 | 0.18267 | 0.38538 |
| **FU97** | 0.01494 | -0.46879 | 0 | 0.98905 | 0 | -0.62381 |
| **OCUM1** | 2.34559 | 0.3332 | 2.11757 | 2.90857 | 0.71951 | 1.99209 |
| **SNU216** | 0.42228 | -0.57458 | 0.23313 | 2.14823 | 0.62168 | 1 .18034 |

The results are shown in FIG. 8, 63 compounds showing toxicity (< 50% survival rate) in four or more EMT-selective gastric cancer cell lines (EMT-selective) were indicated in cyan, and the other compounds (n = 1,282) were marked as pink.

In addition, the value of the Average Area Under the Viability Curve (AUC) of 63 compounds showing toxicity (<50% survival rate) in EMT-selective gastric cancer cell lines that are marked as cyan in FIG. 8 is compared with other gastric cancer cell lines (non-SEM subtype cell lines) to show the result in FIG. 9 and Tables 4-12.

The 63 compounds are niclosamide (Niclocide), TAE684 (NVP-TAE684), MLN2238, Bortezomib (Velcade), MLN9708, Carfilzomib (PR-171), crystal violet, Cetylpyridinium Hydrochloride, Alexidine HCl, WP1130, Zinc pyrithione, Bay 11-7082 (Bay 11-7821), Dronedarone HCl (Multaq), Penfluridol, Thonzonium Bromide, LDN193189, Obatoclax mesylate (GX15-070), LY2603618 (IC-83), Terfenadine, Vorinostat (SAHA), PCI-24781, AR-42 (HDAC-42), Belinostat (PXD101), Trichostatin A (TSA), CUDC-101, SB939 (Pracinostat), JNJ-26481585, Fenbendazole (Panacur), BKM120 (NVP-BKM120), YM155, Mitoxantrone Hydrochloride, Teniposide (Vumon), Doxorubicin (Adriamycin), Daunorubicin HCl (Daunomycin HCl), Triptolide, PIK-75, Ouabain, Topotecan HCl, Camptothecin, Gemcitabine (Gemzar), Fludarabine phosphate (Fludara), GSK2126458, AZD7762, Torin 2, INK128 (MLN0128), BEZ235 (NVP-BEZ235), Neratinib (HKI-272), AT9283, Danusertib (PHA-739358), Oxybendazole, Nocodazole, BI6727 (Volasertib), BI2536, Vincristine, Vinblastine, Epothilone A, Cephalomannine, Paclitaxel (Taxol), Cabazitaxel (Jevtana), SB743921, Ispinesib (SB-715992), or APO866 (FK866).

As a result, according to the experimental results, a total of 63 compounds, among total of 1,345 pharmaceutical compounds, exhibiting toxicity were 63 compounds, and these compounds are expected to exhibit toxicity by specifically inhibiting or competitively inhibiting the expression process of the 19 genes, which are more expressed in SEM-based gastric cancer, or the ATP protein-expressing protein in which the gene is expressed, and thus these compounds are specifically effective in the treatment and prevention of SEM-type gastric cancer. In addition, among the compounds of Table 5 and FIG. 9, it has a low AUC norm value that does not exceed 1 in the SEM-type gastric cancer cell line, and has a high AUC value in the non-SEM-type gastric cancer cell line BEZ235 ^{®}, Drosnetherone ^{®}, PIK-75 alkene, THORIZZIUM BROMIDE ^{®}, Cetylpyridinium Hydrochloride ^{®}, LDN193189 ^{®}, OBATOXCLAX ^{®} Mesylate, WP1130 ^{®}, AT9283 ^{®}, LY2603618 ^{®}, Oubain ^{®}, PYRITHIONE ZINC, Torin ^{®} 2, YM155 ^{®}, BKM120 ^{®}, Crystal Violet, Fenbendazole ^{®}, GSK2126458 ^{®}, The composition was found to be specifically effective in the treatment and prevention of gastric cancer in the type of SEM-based gastric cancer, and among them, it was confirmed that, among them, a large negative AUC norm value in SEM-based gastric cancer, or a negative AUC ∞ value in all SEM-type gastric cancer, BEZ235 ^{®}, AT9283 ^{®}, LY2603618 ^{®}, Oubain ^{®}, Torin ^{®} 2, YM155 ^{®}, It was confirmed that GSK2126458 was specifically effective in the treatment and prevention of lower-level gastric cancer.

### [Example 5] Confirmation of Genetic Dependency of ATP-Binding Proteins - siRNA (Small Interfering RNA) Viaibility Screening

### 1. Specific Experimental Method

In order to confirm the genetic dependence of ATP-binding proteins, loss-of-function screening was performed using a small interfering RNA (siRNA). siRNA (Santa Cruz Biotechnology, INC) prepared by pooling three target specific 19-25 nucleotide was transfactioned into non-SEM^{®} subtype cell lines (AGS^{®}, MKN45^{®}, SNU216^{®} and YCC7) and SEM^{®} subtype cell lines (Hs746T, SK4^{®}, SNU484^{®}, SNU668^{®}, SNU1750 and YC11), followed by measured cell viability using CellTiter 96^{®} Aqueous One Solution Cell Proliferation Assay (MTS, Promega corporation). Each cell line (2 × 10³/100 ul) was seeded into a transparent 96-well plate, and then the target siRNA was transfactioned using Trans-IT X2 Dynamic Delivery System (Mirus Bio), 20 uL of MTS solution was dispensed after 72 hours, and cultured for 3 hours at 37 °C 5% CO₂ incubator (HERAcell 150 l, Thermo Scientific). Colorimetric assay was carried out at a wavelength of 490 nm using VersaMax microplate reader (ELISA, Molecular Devices), and the results are shown in Table 13 below.

**TABLE 13**

| | **Z-score** | | | | | | | | | |
|---|---|---|---|---|---|---|---|---|---|---|
| | **AGS** | **MKN45** | **SNU216** | **YCC7** | **Hs746T** | **SK4** | **SNU484** | **SNU668** | **SNU1750** | **YCC11** |
| **ASS1** | 1.4353 | 0.0164 | 0.2469 | -0.4191 | -1.5979 | -0.5827 | 1.8193 | 0.0728 | -0.4517 | -0.5393 |
| **CYB5B** | 0.4391 | -0.1577 | -0.1528 | 0.0778 | -1.2408 | 0.1783 | -0.5746 | -0.2937 | 2.4868 | -0.7624 |
| **GANAB** | 0.8969 | 0.2775 | 0.8823 | -1.9661 | 0.2623 | -1.6324 | 0.1894 | 0.2813 | 0.7911 | 0.0178 |
| **HAT1** | 1.6937 | 0.5635 | 0.2865 | 0.5334 | -1.1549 | -0.2828 | -1.8983 | 0.3769 | 0.3157 | -0.4338 |
| **HNRNPAO** | 2.4017 | 0.1122 | 0.1994 | 0.6976 | -0.3799 | -0.3366 | -0.5564 | -0.3435 | -0.4514 | -1.3431 |
| **HNRNPA2B1** | 1.4951 | 0.6497 | 0.2122 | 1.0420 | -1.6198 | 0.7558 | -0.8532 | -0.0806 | -0.8196 | -0.7815 |
| **MARCKS** | 0.6605 | 0.2559 | 0.2306 | -0.7722 | 2.1508 | -1.1570 | -0.2485 | 0.3855 | -1.2727 | -0.2328 |
| **PNPO** | 1.4261 | 0.2735 | 0.1512 | -0.0477 | 1.4400 | -1.9548 | -0.0704 | -0.0551 | -0.9604 | -0.2024 |
| **RANBP1** | -0.6849 | 0.2522 | 0.2322 | -0.7360 | 0.9011 | -0.7391 | 2.1902 | -0.3308 | -1.2562 | 0.1713 |
| **REXO2** | 1.2394 | 0.0109 | 0.9814 | 0.1512 | -0.8119 | -1.4628 | 1.2042 | -0.0969 | 0.2526 | -1.4681 |
| **TPM2** | 2.0271 | 0.7311 | 0.6557 | -0.1702 | -1.0640 | -0.9679 | -0.9236 | 0.2923 | -0.8908 | 0.3104 |
| **TXNDC17** | 0.5487 | 1.0893 | 0.0004 | 0.2229 | 0.5339 | -2.0250 | 0.5687 | -0.5413 | 0.8924 | -1.2901 |
| **YWHAZ** | -0.3250 | 0.9955 | 0.9389 | 0.3824 | 0.6076 | -2.4580 | 0.0131 | -0.2470 | 0.4931 | -0.4006 |

### 2. Conclusion

Since the cell viability has a greater genetic dependency with the corresponding gene, as shown in Table 4, when the genes were knocked down as shown in Table 4, it was confirmed that the genes tend to lower cell viability more in SEM cell lines than non-SEM cell lines, and thus the genes were found to have genetic dependence specifically on SEM subtype.

Although the present invention has been described in detail above, the scope of the present invention is not limited thereto, and it would be obvious to a person skilled in the art that various modifications and variations are possible without departing from the technical spirit of the present invention as set forth in the claims.

## Claims

1. A composition for diagnosing gastric cancer or predicting a prognosis comprising:
a material for measuring the expression level of an ATP affinity protein or a gene encoding an ATP affinity protein.

2. The composition of claim 1 for diagnosing gastric cancer or predicting a prognosis, wherein the gene encoding the ATP affinity protein comprises mRNA of at least one gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB and PSMD9.

3. The composition of claim 1 for diagnosing gastric cancer or predicting a prognosis, wherein the ATP affinity protein is a protein selected from the group consisting of Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB and 26S proteasome non-ATPase regulatory subunit 9.

4. The composition of claim 2 for diagnosing gastric cancer or predicting a prognosis, wherein the level of mRNA of the gene encoding the ATP affinity protein is measured by a method selected from the group consisting of RT-PCR, competitive RT-PCR, RNase protection method, northern blotting, DNA chip technology assay, MBD-seq:Methylated DNA binding domain sequencing analysis method and Reduced representation bisulfite sequencing (RRBS) analysis method.

5. The composition of claim 3 for diagnosing gastric cancer or predicting a prognosis, wherein the ATP affinity protein expression level is measured by western blotting, enzyme-linked immunosorbent assay (ELISA), a radioimmunoassay (RIA), a radioimmunodiffusion, Ouchattery immunodiffusion method, a rocket immunoelectrophoresis, immunohistochemical staining, Immunoprecipitation assay, Complement Fixation Assay, Immunofluorescence, Immunochromatography, fluorescence-activated cell sorting analysis (FACS) or protein chip technology assay.

6. The composition according to any one of claims 1 to 5, wherein the gastric cancer is stem-like, epithelial-to-mesenchymal transition and mesenchymal stem-like subunit gastric cancer.

7. A kit for diagnosing gastric cancer or predicting prognosis, comprising the composition of claim 6.

8. The kit of claim 7 for diagnosing gastric cancer or predicting prognosis, wherein the kit is a reverse transcription polymerase chain reaction (RT-PCR) kit, a DNA chip kit, an enzyme-linked immunosorbent assay (ELISA) kit, or a protein chip kit.

9. The kit of claims 7 or 8 for diagnosing gastric cancer or predicting prognosis, wherein the gastric cancer is a stem-like, epithelial-to-mesenchymal transition and mesenchymal subunit gastric cancer.

10. A method for providing information for diagnosing gastric cancer or predicting prognosis comprising:
(a) measuring mRNA level of a gene encoding an ATP affinity protein or an expression level of an ATP-affinity protein in a biological sample; and
(b) comparing the measurement result from step (a) with the mRNA level of the gene encoding the ATP affinity protein or the expression level of the ATP affinity protein or of the control sample.

11. The method of claim 10 for providing information for diagnosing gastric cancer or predicting prognosis, wherein the gene encoding the ATP affinity protein comprises mRNA of at least one gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB and PSMD9.

12. The method of claim 10 for providing information for diagnosing gastric cancer or predicting prognosis, wherein the ATP affinity protein is a protein selected from the group consisting of Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB and 26S proteasome non-ATPase regulatory subunit 9.

13. The method of claim 11 for providing information for diagnosing gastric cancer or predicting prognosis, wherein the level of mRNA of the gene encoding the ATP affinity protein is measured by a method selected from the group consisting of RT-PCR, competitive RT-PCR, RNase protection method, northern blotting, DNA chip technology assay, MBD-seq:Methylated DNA binding domain sequencing analysis method and Reduced representation bisulfite sequencing (RRBS) analysis method.

14. The method of claim 12 for providing information for diagnosing gastric cancer or predicting prognosis, wherein the ATP affinity protein expression level is measured by western blotting, enzyme-linked immunosorbent assay (ELISA), a radioimmunoassay (RIA), a radioimmunodiffusion, Ouchattery immunodiffusion method, a rocket immunoelectrophoresis, immunohistochemical staining, Immunoprecipitation assay, Complement Fixation Assay, Immunofluorescence, Immunochromatography, fluorescence-activated cell sorting analysis (FACS), or protein chip technology assay.

15. The method of any one of claims 10 to 14 for providing information for diagnosing gastric cancer or predicting prognosis, wherein the gastric cancer is a stem-like, epithelial-to-mesenchymal transition and mesenchymal subunit gastric cancer.

16. An device for diagnosing gastric cancer or predicting prognosis comprises:
a measuring unit that measures the mRNA level of a gene encoding an ATP affinity protein present in a biological sample or the expression level of an ATP affinity protein; and
a comparison unit comparing the measurement results with the mRNA level of the gene encoding the ATP affinity protein or the expression level of the ATP affinity protein in the control sample.

17. The device of claim 16 for diagnosing gastric cancer or predicting prognosis, wherein the gene encoding the ATP affinity protein comprises mRNA of at least one gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB and PSMD9.

18. The device of claim 16 for diagnosing gastric cancer or predicting prognosis, wherein the ATP affinity protein is a protein selected from the group consisting of Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB and 26S proteasome non-ATPase regulatory subunit 9.

19. The device of claim 16 for diagnosing gastric cancer or predicting prognosis, wherein the gastric cancer is a stem-like, epithelial-to-mesenchymal transition and mesenchymal subunit gastric cancer.

20. A method of screening an agent for preventing or treating gastric cancer comprising:
(a) treating gastric cancer cells with a gastric cancer treatment a candidate substance;
(b) measuring mRNA level of a gene encoding an ATP affinity protein or an expression level of an ATP-affinity protein in the isolated gastric cancer cells treated with a candidate substance in step (a); and
(c) determining that the candidate material can be used as an agent for preventing or treating gastric cancer when if the mRNA level of a gene encoding an ATP affinity protein or an expression level of an ATP-affinity protein measured in step (b) is lower than that of the isolated gastric cancer cells in which the candidate material is not treated.

21. The method of claim 20 for screening an agent for preventing or treating gastric cancer, wherein the gene encoding the ATP affinity protein comprises mRNA of at least one gene selected from the group consisting of CAST, EEF1D, HNRNPA2B1, LIG1, MARCKS, MTA2, PFAS, PNPO, REXO2, YWHAZ, HNRNPAO, TPM2, TXNDC17, ASS1, RANBP1, HAT1, CYB5B, GANAB and PSMD9.

22. The method of claim 20 for screening an agent for preventing or treating gastric cancer, wherein the ATP affinity protein is a protein selected from the group consisting of Calpastatin, Elongation factor 1-delta, Heterogeneous nuclear ribonucleoproteins A2/B1, DNA ligase 1, Myristoylated alanine-rich C-kinase substrate, Metastasis-associated protein MTA2, Phosphoribosylformylglycinamidine synthase, Pyridoxine-5'-phosphate oxidase, Oligoribonuclease, mitochondrial, 14-3-3 protein zeta/delta, Heterogeneous nuclear ribonucleoproteins A0, Tropomyosin beta chain, Thioredoxin domain-containing protein 17, Argininosuccinate synthase, Ran-specific GTPase-activating protein, Histone acetyltransferase type B catalytic subunit, Cytochrome b5 type B, Neutral alpha-glucosidase AB and 26S proteasome non-ATPase regulatory subunit 9.

23. The method of claim 20 for screening an agent for preventing or treating gastric cancer, wherein the level of mRNA of the gene encoding the ATP affinity protein is measured by a method selected from the group consisting of RT-PCR, competitive RT-PCR, RNase protection method, northern blotting, DNA chip technology assay, MBD-seq:Methylated DNA binding domain sequencing analysis method and Reduced representation bisulfite sequencing (RRBS) analysis method.

24. The method of claim 22 for screening an agent for preventing or treating gastric cancer, wherein the ATP affinity protein expression level is measured by western blotting, enzyme-linked immunosorbent assay (ELISA), a radioimmunoassay (RIA), a radioimmunodiffusion, Ouchattery immunodiffusion method, a rocket immunoelectrophoresis, immunohistochemical staining, Immunoprecipitation assay, Complement Fixation Assay, Immunofluorescence, Immunochromatography, fluorescence-activated cell sorting analysis (FACS), or protein chip technology assay.

25. The method of any one of claims 20 to 24 for screening an agent for preventing or treating gastric cancer, wherein the gastric cancer is a stem-like, epithelial-to-mesenchymal transition and mesenchymal subunit gastric cancer.

26. A pharmaceutical composition for preventing or treating gastric cancer, comprising a substance targeting epithelial-to-mesenchymal transition (EMT) as an active ingredient.

27. The pharmaceutical composition of claim 26 for preventing or treating gastric cancer, wherein the substance targeting epithelial-to-mesenchymal transition (EMT) is Niclosamide(Niclocide), TAE684(NVP-TAE684), MLN2238, Bortezomib(Velcade), MLN9708, Carfilzomib(PR-171), Crystal violet, Cetylpyridinium Chloride, Alexidine HCl, WP1130, Pyrithione zinc, BAY 11-7082(BAY 11-7821), Dronedarone HCl(Multaq), Penfluridol, Thonzonium Bromide, LDN193189, Obatoclax mesylate(GX15-070), LY2603618(IC-83), Terfenadine, Vorinostat(SAHA), PCI-24781, AR-42(HDAC-42), Belinostat(PXD101), Trichostatin A(TSA), CUDC-101, SB939(Pracinostat), JNJ-26481585, Fenbendazole(Panacur), BKM120(NVP-BKM120), YM155, Mitoxantrone Hydrochloride, Teniposide(Vumon), Doxorubicin(Adiriamycin), Daunorubicin HCl(Daunomycin HCl), Triptolide, PIK-75, Ouabain, Topotecan HCl, Camptothecin, Gemcitabine(Gemzar), Fludarabine Phosphate(Fludara), GSK2126458, AZD77662, Torin 2, INK 128(MLN0128), BEZ235(NVP-BEZ235), Neratinib(HKI-272), AT9283, Danusertib(PHA-739358), Oxibendazole, Nocodazole, BI6727(Volasertib), BI2536, Vincristine, Vinblastine, Epothilone A, Cephalomannine, Paclitaxel(Taxol), Cabazitaxel(Jevtana), SB743921, Ispinesib(SB-715992) or APO866(FK866).

28. The pharmaceutical composition according to any one of claims 26 to 27 for preventing or treating gastric cancer, wherein the gastric cancer is a stem-like, epithelial-to-mesenchymal transition and mesenchymal subunit gastric cancer.
